(19) Europäisches Patentamt
European Patent Office
Office européen des brevets

(11) EP 1 466 616 A2

(12) EUROPEAN PATENT APPLICATION

(43) Date of publication:
**13.10.2004 Bulletin 2004/42**

(21) Application number: **04076929.1**

(22) Date of filing: **28.02.2000**

(51) Int Cl.[7]: **A61K 38/17**, A61K 39/395, C07K 14/705, A61P 35/00, A61K 39/00, A61K 48/00, C07K 16/28, G01N 33/68, C12Q 1/68, C12N 15/12

(84) Designated Contracting States:
**AT BE CH CY DE DK ES FI FR GB GR IE IT LI LU MC NL PT SE**
Designated Extension States:
**SI**

(30) Priority: **05.03.1999 GB 9905124**

(62) Document number(s) of the earlier application(s) in accordance with Art. 76 EPC:
**00907633.2 / 1 162 993**

(71) Applicant: **GlaxoSmithKline Biologicals S.A.**
**1330 Rixensart (BE)**

(72) Inventor: **Vinals y de Bassols, C.**
**GlaxoSmithKline Biolog. SA**
**1330 Rixensart (BE)**

(74) Representative: **Riley, Victoria Jayne**
**GlaxoSmithKline,**
**Corporate Intellectual Property CN925.1,**
**980 Great West Road**
**Brentford, Middlesex TW8 9GS (GB)**

Remarks:
This application was filed on 02 - 07 - 2004 as a divisional application to the application mentioned under INID code 62.

(54) **Use of casb616 polypeptides and polynucleotides for cancer treatment**

(57) Use of CASB616 polypeptides and polynucleotides in diagnostics, and vaccines for prophylactic and therapeutic treatment of cancers, particularly colon cancers, autoimmune diseases, and related conditions.

EP 1 466 616 A2

## Description

The present invention relates to methods for using polypeptides and polynucleotides (herein referred to as "CASB616" polypeptide(s) and "CASB616" polynucleotide(s)"), including the treatment of cancer and autoimmune diseases and other related conditions. In a further aspect, the invention relates to methods for identifying agonists and antagonists/inhibitors using the materials provided by the invention, and treating conditions associated with CASB616 polypeptide imbalance with the identified compounds. In a still further aspect, the invention relates to diagnostic assays for detecting diseases associated with inappropriate CASB616 polypeptide activity or levels.

It has been discovered that CASB616 encodes a polypeptide which belongs to the largest family of receptor protein-tyrosine kinases, the EPH and EPH-related receptors. CASB616 is otherwise known as EPHB2 (aliases: EBHB2, , ERK, EPH3, EPHT3, DRT, HEK5) and EPHB2v.

The EPH and EPH-related receptors have been implicated in mediating developmental events, particularly in the nervous system. Receptors in the Eph subfamily typically have a single kinase domain and an extracellular region containing a Cys-rich domain and 2 fibronectin type III repeats. The ligands for Eph receptors have been named ephrins by the Eph Nomenclature Committee (Cell 90: 403-404, 1997; PubMed ID : 9267020). Based on their structures and sequence relationships, ephrins are divided into the ephrin-A (EFNA) class, which are anchored to the membrane by a glycosylphosphatidylinositol linkage, and the ephrin-B (EFNB) class, which are transmembrane proteins. The Eph family of receptors are divided into 2 groups based on the similarity of their extracellular domain sequences and their affinities for binding ephrin-A and ephrin-B ligands. The Eph Nomenclature Committee (1997) proposed that Eph receptors interacting preferentially with ephrin-A proteins be called EphA and Eph receptors interacting preferentially with ephrin-B proteins be called EphB.

Ikegaki *et al*. (Hum. Molec. Genet. 4: 2033-2045, 1995) mapped DRT, the EPHB2 gene, to 1p36.1-p35 by PCR screening of human/rodent somatic cell hybrid panels and by fluorescence in situ hybridization. As the distal end of 1p is often deleted in neuroblastomas, the DRT gene may play a role in neuroblastoma and small cell lung carcinoma (SCLC) tumorigenesis.

By fluorescence in situ hybridization, Saito *et al*. (Genomics 26: 382-384, 1995 PubMed ID : 7601466) demonstrated that the ERK gene is located in chromosomal region 1p36.1. They showed that the homologous genes are located on mouse 4D2.2-D3 and rat 5q36.13, both of which are regions with conserved linkage homology to human chromosome 1p.

The invention relates to the use of CASB616 polypeptides and polynucleotides as described in greater detail below. The invention relates especially to the use of CASB616 having the nucleotide and amino acid sequences set out in SEQ ID NO:1 or 3 and SEQ ID NO:2 or 4 respectively.

The invention further relates to uses of polynucleotides and polypeptides which have at least 85% identity, preferably at least 90% identity, more preferably at least 95% identity, most preferably at least 97-99% or exact identity to the sequences identified in SEQ ID NO:1 or 3 and SEQ ID NO:2 or 4.

CASB616 polypeptides and polynucleotides are believed to be important immunogens for specific prophylactic or therapeutic immunization against tumours, because they are specifically expressed or highly over-expressed in tumours compared to normal cells and can thus be targeted by antigen-specific immune mechanisms leading to the destruction of the tumour cell. They can also be used to diagnose the occurrence of tumour cells. Furthermore, their inappropriate expression in certain circumstances can cause an induction of autoimmune, inappropriate immune responses, which could be corrected through appropriate vaccination using the same polypeptides or polynucleotides. In this respect the most important biological activities to our purpose are the antigenic and immunogenic activities of the polypeptide of the present invention. A polypeptide of the present invention may also exhibit at least one other biological activity of a CASB616 polypeptide, which could qualify it as a target for therapeutic or prophylactic intervention different from that linked to the immune response.

In a first aspect of the invention there are provided uses for CASB616 polypeptides as well as biologically, diagnostically, prophylactically, clinically or therapeutically useful variants thereof, and compositions comprising the same.

The present invention further provides uses for:

(a) an isolated polypeptide which comprises an amino acid sequence which has at least 85% identity, more preferably at least 90% identity, yet more preferably at least 95% identity, most preferably at least 97-99% or exact identity, to that of SEQ ID NO:2 or 4;

(b) a polypeptide encoded by an isolated polynucleotide comprising a polynucleotide sequence which has at least 85% identity, more preferably at least 90% identity, yet more preferably at least 95% identity, even more preferably at least 97-99% or exact identity to SEQ ID NO:1 or 3 over the entire length of SEQ ID NO:1 or 3 respectively; or

(c) a polypeptide encoded by an isolated polynucleotide comprising a polynucleotide sequence encoding a polypeptide which has at least 85% identity, more preferably at least 90% identity, yet more preferably at least 95% identity, even more preferably at least 97-99% or exact identity, to the amino acid sequence of SEQ ID NO: 2 or 4.

The invention also provides uses for immunogenic fragments of a CASB616 polypeptide, that is, a contiguous portion of the CASB616 polypeptide which has the same or substantially the same immunogenic activity as the polypeptide comprising the amino acid sequence of SEQ ID NO:2 or 4. That is to say, the fragment (if necessary when coupled to a carrier) is capable of raising an immune response which recognises the CASB616 polypeptide. Such an immunogenic fragment may include, for example, the CASB616 polypeptide lacking an N-terminal leader sequence, and/or a transmembrane domain and.or a C-terminal anchor domain. In a preferred aspect the immunogenic fragment of CASB616 according to the invention comprises substantially all of the extracellular domain of a polypeptide which has at least 85% identity, preferably at least 90% identity, more preferably at least 95% identity, most preferably at least 97-99% identity, more preferably at least more preferably at least 95% identity, most preferably at least 97-99% identity, to that of SEQ ID NO:2 or 4 over the entire length of SEQ ID NO:2 or 4 respectively.

A fragment is a polypeptide having an amino acid sequence that is entirely the same as part but not all of any amino acid sequence of any polypeptide of the invention. As with CASB616 polypeptides, fragments may be "free-standing", or comprised within a larger polypeptide of which they form a part or region, most preferably as a single continuous region in a single larger polypeptide.

Preferred fragments include, for example, truncation polypeptides having a portion of an amino acid sequence of SEQ ID NO:2 or 4 or of a variant thereof, such as a continuous series of residues that includes an amino- and/or carboxyl-terminal amino acid sequence. Degradation forms of the polypeptides of the invention produced by or in a host cell, are also preferred. Further preferred are fragments characterized by structural or functional attributes such as fragments that comprise beta-barrels, alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions.

Further preferred fragments include an isolated polypeptide comprising an amino acid sequence having at least 15, 20, 30, 40, 50 or 100 contiguous amino acids from the amino acid sequence of SEQ ID NO:2 or 4, or an isolated polypeptide comprising an amino acid sequence having at least 15, 20, 30, 40, 50 or 100 contiguous amino acids truncated or deleted from the amino acid sequence of SEQ ID NO:2 or 4.

Particularly preferred are variants in which several, 5-10, 1-5, 1-3, 1-2 or 1 amino acids are substituted, deleted, or added in any combination.

The polypeptides, or immunogenic fragments, for use in the invention may be in the form of the "mature" protein or may be a part of a larger protein such as a precursor or a fusion protein. It is often advantageous to include an additional amino acid sequence which contains secretory or leader sequences, pro-sequences, sequences which aid in purification such as multiple histidine residues, or an additional sequence for stability during recombinant production. Furthermore, addition of exogenous polypeptide or lipid tail or polynucleotide sequences to increase the immunogenic potential of the final molecule is also considered.

In one aspect, the invention relates to the use of genetically engineered soluble fusion proteins comprising a polypeptide of the present invention, or a fragment thereof, and various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. In a particular embodiment, the Fc part can be removed simply by incorporation of a cleavage sequence which can be cleaved with blood clotting factor Xa.

Examples of fusion protein technology can be found in International Patent Application Nos. WO94/29458 and WO94/22914.

The proteins may be chemically conjugated, or expressed as recombinant fusion proteins allowing increased levels to be produced in an expression system as compared to non-fused protein. The fusion partner may assist in providing T helper epitopes (immunological fusion partner), preferably T helper epitopes recognised by humans, or assist in expressing the protein (expression enhancer) at higher yields than the native recombinant protein. Preferably the fusion partner will be both an immunological fusion partner and expression enhancing partner.

Fusion partners include protein D from *Haemophilus influenzae* and the non-structural protein from influenza virus, NS1 (hemagglutinin). Another fusion partner is the protein known as LytA. Preferably the C terminal portion of the molecule is used. Lyta is derived from *Streptococcus pneumoniae* which synthesize an N-acetyl-L-alanine amidase LytA, (coded by the lytA gene {Gene, 43 (1986) page 265-272}) an autolysin that specifically degrades certain bonds in the peptidoglycan backbone. The C-terminal domain of the LytA protein is responsible for the affinity to the choline or to some choline analogues such as DEAE. This property has been exploited for the development of *E.coli* C- LytA expressing plasmids useful for expression of fusion proteins. Purification of hybrid proteins containing the C- LytA fragment at its amino terminus has been described {Biotechnology: 10, (1992) page 795-798}. It is possible to use the repeat portion of the LytA molecule found in the C terminal end starting at residue 178, for example residues 188 - 305.

The present invention also includes variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr.

Polypeptides for use in the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

In a further aspect, the present invention relates to uses for polynucleotides that encode CASB616 polypeptides, particularly polynucleotides that encode the polypeptide herein designated CASB616, for use in or in preparation of the vaccine compositions described herein.

In a particularly preferred embodiment the polynucleotide comprises a region encoding CASB616 polypeptides comprising a sequence set out in SEQ ID NO:1 or 3 which includes a full length gene, or a variant thereof.

Using the information provided herein, such as a polynucleotide sequence set out in SEQ ID NO:1 or 3 a polynucleotide of the invention encoding CASB616 polypeptide may be obtained using standard cloning and screening methods, from a cDNA library derived from mRNA in cells of human colon cancer, lung cancer, uterine cancer and fetal tissues. Suitable techniques are described by Maniatis, T., Fritsch, E.F. and Sambrook et al., *MOLECULAR CLONING, A LABORATORY MANUAL*, 2nd Ed.; Cold Spring Harbor Laboratory Press, Cold Spring Harbor, New York (1989). Polynucleotides as described in the invention can also be obtained from natural sources such as genomic DNA libraries or can be synthesized using well known and commercially available techniques.

Moreover, the DNA sequence set out in SEQ ID NO:1 or 3 contains an open reading frame encoding a protein having about the number of amino acid residues set forth in SEQ ID NO:2 or 4 with a deduced molecular weight that can be calculated using amino acid residue molecular weight values well known to those skilled in the art.

The polynucleotide of SEQ ID NO:1, between the start codon at nucleotide number 105 and the stop codon which begins at nucleotide number 3066 of SEQ ID NO:1, encodes the polypeptide of SEQ ID NO:2.

The polynucleotide of SEQ ID NO:3, between the start codon at nucleotide number 26 and the stop codon which begins at nucleotide number 3191 of SEQ ID NO:3, encodes the polypeptide of SEQ ID NO:4.

In a further aspect, the present invention provides uses for an isolated polynucleotide comprising or consisting of:

(a) a polynucleotide sequence which has at least 85% identity, more preferably at least 90% identity, yet more preferably at least 95% identity, even more preferably at least 97-99% or exact identity to SEQ ID NO:1 or 3 over the entire length of SEQ ID NO:1 or 3; or
(b) a polynucleotide sequence encoding a polypeptide which has at least 85% identity, more preferably at least 90% identity, yet more preferably at least 95% identity, even more preferably at least 97-99% or 100% exact, to the amino acid sequence of SEQ ID NO:2 or 4 over the entire length of SEQ ID NO:2 or 4.

A polynucleotide encoding a polypeptide for use in the present invention, may be obtained by a process which comprises the steps of screening an appropriate library under stringent hybridization conditions (for example, using a temperature in the range of 45 - 65°C and an SDS concentration from 0.1 - 1%) with a labeled or detectable probe consisting of or comprising the sequence of SEQ ID NO:1 or 3 or a fragment thereof; and isolating a full-length gene and/or genomic clones containing said polynucleotide sequence.

The invention provides uses for a polynucleotide sequence identical over its entire length to a coding sequence (open reading frame) in SEQ ID NO:1 or 3. Also provided by the invention are uses for a coding sequence for a mature polypeptide or a fragment thereof, by itself as well as a coding sequence for a mature polypeptide or a fragment in reading frame with another coding sequence, such as a sequence encoding a leader or secretory sequence, a pre-, or pro- or prepro-protein sequence, or other fusion peptide portions. The polynucleotide may also contain at least one non-coding sequence, including for example, but not limited to at least one non-coding 5' and 3' sequence, such as the transcribed but non-translated sequences, termination signals (such as rho-dependent and rho-independent termination signals), ribosome binding sites, Kozak sequences, sequences that stabilize mRNA, introns, and polyadenylation signals. The polynucleotide sequence may also comprise additional coding sequence encoding additional amino acids. For example, a marker sequence that facilitates purification of the fused polypeptide can be encoded. In certain embodiments of the invention, the marker sequence is a hexa-histidine peptide, as provided in the pQE vector (Qiagen, Inc.) and described in Gentz *et al*., *Proc. Natl. Acad. Sci., USA 86:* 821-824 (1989), or an HA peptide tag (Wilson *et al., Cell 37:* 767 (1984), both of which may be useful in purifying polypeptide sequence fused to them. Polynucleotides for use with the invention also include, but are not limited to, polynucleotides comprising a structural gene and its naturally associated sequences that control gene expression.

The nucleotide sequence encoding CASB616 polypeptide of SEQ ID NO:2 or 4 may be identical to the polypeptide encoding sequence contained in nucleotides 105 to 3068 of SEQ ID NO:1, or the polypeptide encoding sequence contained in nucleotides 26 to 3193 of SEQ ID NO:3, respectively. Alternatively it may be a sequence, which as a result of the redundancy (degeneracy) of the genetic code, also encodes the polypeptide of SEQ ID NO:2 or 4.

The term "polynucleotide encoding a polypeptide" as used herein encompasses polynucleotides that include a sequence encoding a polypeptide of the invention, particularly a polypeptide having an amino acid sequence set out in SEQ ID NO:2 or 4. The term also encompasses polynucleotides that include a single continuous region or discontinuous regions encoding the polypeptide (for example, polynucleotides interrupted by integrated phage, an integrated insertion sequence, an integrated vector sequence, an integrated transposon sequence, or due to RNA editing or genomic DNA reorganization) together with additional regions, that also may contain coding and/or non-coding sequences.

The invention further relates to variants of the polynucleotides described herein that encode variants of a polypeptide having a deduced amino acid sequence of SEQ ID NO:2 or 4. Fragments of polynucleotides of the invention may be used, for example, to synthesize full-length polynucleotides of the invention.

Further particularly preferred embodiments are polynucleotides encoding CASB616 variants, that have the amino acid sequence of CASB616 polypeptide of SEQ ID NO:2 or 4 in which several, a few, 5 to 10, 1 to 5, 1 to 3, 2, 1 or no amino acid residues are substituted, modified, deleted and/or added, in any combination. Especially preferred among these are silent substitutions, additions and deletions, that do not alter the properties and activities of CASB616 polypeptide.

Further preferred for use in the invention are polynucleotides that are at least 85% identical over their entire length to a polynucleotide encoding CASB616 polypeptide having an amino acid sequence set out in SEQ ID NO:2 or 4, and polynucleotides that are complementary to such polynucleotides. Alternatively, most highly preferred are polynucleotides that comprise a region that is at least 90% identical over its entire length to a polynucleotide encoding CASB616 polypeptide and polynucleotides complementary thereto. In this regard, polynucleotides at least 95% identical over their entire length to the same are particularly preferred. Furthermore, those with at least 97% are highly preferred among those with at least 95%, and among these those with at least 98% and at least 99% are particularly highly preferred, with at least 99% being the more preferred.

Preferred embodiments are polynucleotides encoding polypeptides that retain substantially the same biological function or activity as the mature polypeptide encoded by a DNA of SEQ ID NO:1 or 3.

In accordance with certain preferred embodiments of this invention there are provided uses of polynucleotides that hybridize, particularly under stringent conditions, to CASB616 polynucleotide sequences, such as the polynucleotides in SEQ ID NO:1 or 3.

The invention further relates to uses of polynucleotides that hybridize to the polynucleotide sequences provided herein. In this regard, the invention especially relates to uses of polynucleotides that hybridize under stringent conditions to the polynucleotides described herein. As herein used, the terms "stringent conditions" and "stringent hybridization conditions" mean hybridization occurring only if there is at least 95% and preferably at least 97% identity between the sequences. A specific example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml of denatured, sheared salmon sperm DNA, followed by washing the hybridization support in 0.1x SSC at about 65°C. Hybridization and wash conditions are well known and exemplified in Sambrook, *et al.*, Molecular Cloning: A Laboratory Manual, Second Edition, Cold Spring Harbor, N.Y., (1989), particularly Chapter 11 therein. Solution hybridization may also be used with the polynucleotide sequences provided by the invention.

A coding region of a CASB616 gene may be isolated by screening using a DNA sequence provided in SEQ ID NO:1 or 3 to synthesize an oligonucleotide probe. A labeled oligonucleotide having a sequence complementary to that of a gene of the invention is then used to screen a library of cDNA, genomic DNA or mRNA to determine which members of the library the probe hybridizes to.

There are several methods available and well known to those skilled in the art to obtain full-length DNAs, or extend short DNAs, for example those based on the method of Rapid Amplification of cDNA ends (RACE) (see, for example, Frohman, *et al., PNAS USA 85:* 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end.

Nucleic acid amplification (PCR) is then carried out to amplify the "missing" 5' end of the DNA using a combination of gene specific and adaptor specific oligonucleotide primers. The PCR reaction is then repeated using "nested" primers, that is, primers designed to anneal within the amplified product (typically an adaptor specific primer that anneals further 3' in the adaptor sequence and a gene specific primer that anneals further 5' in the selected gene sequence).

The products of this reaction can then be analyzed by DNA sequencing and a full-length DNA constructed either by joining the product directly to the existing DNA to give a complete sequence, or carrying out a separate full-length PCR using the new sequence information for the design of the 5' primer.

The invention also provides uses for polynucleotides that encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

A precursor protein, having a mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

Recombinant polypeptides of the present invention may be prepared by processes well known in the art from genetically engineered host cells comprising expression systems. Accordingly, in a further aspect, the present invention relates to an expression system which comprises a polynucleotide of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., Basic Methods in Molecular Biology (1986) and Sambrook *et al*., Molecular Cloning: A Laboratory Manual, 2nd Ed., Cold Spring Harbor Laboratory Press, Cold Spring Harbor, N.Y. (1989). Preferred such methods include, for instance, calcium phosphate transfection, DEAE-dextran mediated transfection, transvection, microinjection, cationic lipid-mediated transfection, electroporation, transduction, scrape loading, ballistic introduction or infection.

Preferably the proteins of the invention are coexpressed with thioredoxin in trans (TIT). Coexpression of thioredoxin in trans versus in cis is preferred to keep antigen free of thioredoxin without the need for protease. Thioredoxin coexpression eases the solubilisation of the proteins of the invention. Thioredoxin coexpression has also a significant impact on protein purification yield, on purified-protein solubility and quality.

Representative examples of appropriate hosts include bacterial cells, such as *Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector which is able to maintain, propagate or express a polynucleotide to produce a polypeptide in a host may be used. The appropriate nucleotide sequence may be inserted into an expression system by any of a variety of well-known and routine techniques, such as, for example, those set forth in Sambrook *et al*., Molecular Cloning, A Laboratory Manual (supra). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

The expression system may also be a recombinant live microorganism, such as a virus or bacterium. The gene of interest can be inserted into the genome of a live recombinant virus or bacterium. Inoculation and *in vivo* infection with this live vector will lead to *in vivo* expression of the antigen and induction of immune responses. Viruses and bacteria used for this purpose are for instance: poxviruses (e.g; vaccinia, fowlpox, canarypox), alphaviruses (Sindbis virus, Semliki Forest Virus, Venezuelian Equine Encephalitis Virus), adenoviruses, adeno-associated virus, picornaviruses (poliovirus, rhinovirus), herpesviruses (varicella zoster virus, etc), Listeria, Salmonella, Shigella, BCG. These viruses and bacteria can be virulent, or attenuated in various ways in order to obtain live vaccines. Such live vaccines also form part of the invention.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, ion metal affinity chromatography (IMAC) is employed for purification. Well known techniques for refolding proteins may be employed to regenerate active conformation when the polypeptide is denatured during intracellular synthesis, isolation and or purification.

Another important aspect of the invention relates to a method for inducing , re-inforcing or modulating an immunological response in a mammal which comprises inoculating the mammal with a fragment or the entire polypeptide or polynucleotide of the invention, adequate to produce antibody and/or T cell immune response for prophylaxis or for therapeutic treatment of cancer and autoimmune disease and related conditions. Yet another aspect of the invention relates to a method of inducing, re-inforcing or modulating immunological response in a mammal which comprises, delivering a polypeptide of the present invention *via* a vector or cell directing expression of the polynucleotide and coding for the polypeptide *in vivo* in order to induce such an immunological response to produce immune responses for prophylaxis or treatment of said mammal from diseases.

A further aspect of the invention relates to an immunological/vaccine formulation (composition) which, when introduced into a mammalian host, induces, re-inforces or modulates an immunological response in that mammal to a polypeptide of the present invention wherein the composition comprises a polypeptide or polynucleotide of the invention or an immunological fragment thereof as herein before defined.The vaccine formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use.

A further aspect of the invention relates to the immunization of a mammal by administration of antigen presenting cells modified by *in vitro* loading with part or the entire polypeptide of the present invention or a molecule comprising the polypeptide of the present invention and administered *in vivo* in an immunogenic way. Alternatively, antigen presenting cells can be transfected *in vitro* with a vector containing a fragment or the entire polynucleotide of the present invention or a molecule comprising the polynucleotide of the present invention, such as to express the corresponding polypeptide, and administered *in vivo* in an immunogenic way.

The vaccine formulation of the invention may also include adjuvant systems for enhancing the immunogenicity of the formulation. Preferably the adjuvant system raises preferentially a TH1 type of response.

An immune response may be broadly distinguished into two extreme catagories, being a humoral or cell mediated immune responses (traditionally characterised by antibody and cellular effector mechanisms of protection respectively). These categories of response have been termed TH1-type responses (cell-mediated response), and TH2-type immune responses (humoral response).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgG1 type antibodies. TH2-type immune responses are characterised by the generation of a broad range of immunoglobulin isotypes including in mice IgG1, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman (*Mosmann, T.R. and Coffman, R.L. (1989) TH1 and TH2 cells: different patterns of lymphokine secretion lead to different functional properties. Annual Review of Immunology, 7, p145-173*). Traditionally, TH1-type responses are associated with the production of the INF-γ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH1:TH2 balance of the immune response after a vaccination or infection includes direct measurement of the production of TH1 or TH2 cytokines by T lymphocytes *in vitro* after restimulation with antigen, and/or the measurement of the IgG1:IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

Adjuvants which are capable of preferential stimulation of the TH1 cell response are described in International Patent Application No. WO 94/00153 and WO 95/17209.

3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem, Montana. A preferred form of 3 De-O-acylated monophosphoryl lipid A is disclosed in European Patent 0 689 454 B1 (SmithKline Beecham Biologicals SA).

Preferably, the particles of 3D-MPL are small enough to be sterile filtered through a 0.22micron membrane (European Patent number 0 689 454).

3D-MPL will be present in the range of 10µg - 100µg preferably 25-50µg per dose wherein the antigen will typically be present in a range 2-50µg per dose.

Another preferred adjuvant comprises QS21, an Hplc purified non-toxic fraction derived from the bark of Quillaja Saponaria Molina. Optionally this may be admixed with 3 De-O-acylated monophosphoryl lipid A (3D-MPL), optionally together with an carrier.

The method of production of QS21 is disclosed in US patent No. 5,057,540.

Non-reactogenic adjuvant formulations containing QS21 have been described previously (WO 96/33739). Such formulations comprising QS21 and cholesterol have been shown to be successful TH1 stimulating adjuvants when formulated together with an antigen.

Further adjuvants which are preferential stimulators of TH1 cell response include immunomodulatory oligonucleotides, for example unmethylated CpG sequences as disclosed in WO 96/02555.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D-MPL: QS21.

Preferably a carrier is also present in the vaccine composition according to the invention. The carrier may be an oil in water emulsion, or an aluminium salt, such as aluminium phosphate or aluminium hydroxide.

A preferred oil-in-water emulsion comprises a metabolisible oil, such as squalene, alpha tocopherol and Tween 80. In a particularly preferred aspect the antigens in the vaccine composition according to the invention are combined with QS21 and 3D-MPL in such an emulsion. Additionally the oil in water emulsion may contain span 85 and/or lecithin and/or tricaprylin.

Typically for human administration QS21 and 3D-MPL will be present in a vaccine in the range of 1µg - 200µg, such as 10-100µg, preferably 10µg - 50µg per dose. Typically the oil in water will comprise from 2 to 10% squalene, from 2 to 10% alpha tocopherol and from 0.3 to 3% tween 80. Preferably the ratio of squalene: alpha tocopherol is equal to or less than 1 as this provides a more stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The present invention also provides a polyvalent vaccine composition comprising a vaccine formulation of the invention in combination with other antigens, in particular antigens useful for treating cancers, autoimmune diseases and related conditions. Such a polyvalent vaccine composition may include a TH-1 inducing adjuvant as hereinbefore described.

This invention also relates to the use of polynucleotides, in the form of primers derived from the polynucleotides of the present invention, and of polypeptides, in the form of antibodies or reagents specific for the polypeptide of the present invention, as diagnostic reagents.

The identification of genetic or biochemical markers in blood or tissues that will enable the detection of very early changes along the carcinogenesis pathway will help in determining the best treatment for the patient. Surrogate tumour markers, such as polynucleotide expression, can be used to diagnose different forms and states of cancer. The identification of expression levels of the polynucleotides of the invention will be useful in both the staging of the cancerous disorder and grading the nature of the cancerous tissue. The staging process monitors the advancement of the cancer and is determined on the presence or absence of malignant tissue in the areas biopsied. The polynucleotides of the invention can help to perfect the staging process by identifying markers for the aggresivity of a cancer, for example the presence in different areas of the body. The grading of the cancer describes how closely a tumour resembles normal tissue of its same type and is assessed by its cell morphology and other markers of differentiation. The polynucleotides of the invention can be useful in determining the tumour grade as they can help in the determination of the differentiation status of the cells of a tumour. On the other hand, the polypeptide of the invention can be produced by stroma cells, in which cases, its specific expression or differential expression is a marker of disease conditions.

The diagnostic assays offer a process for diagnosing or determining a susceptibility to cancers, autoimmune disease and related conditions through diagnosis by methods comprising determining from a sample derived from a subject an abnormally decreased or increased level of polypeptide or mRNA. This method of diagnosis is known as differential expression. The expression of a particular gene is compared between a diseased tissue and a normal tissue. A difference between the polynucleotide-related gene, mRNA, or protein in the two tissues is compared, for example in molecular weight, amino acid or nucleotide sequence, or relative abundance, indicates a change in the gene, or a gene which regulates it, in the tissue of the human that was suspected of being diseased.

Decreased or increased expression can be measured at the RNA level. PolyA RNA is first isolated from the two tissues and the detection of mRNA encoded by a gene corresponding to a differentially expressed polynucleotide of the invention can be detected by, for example, in situ hybridization in tissue sections, reverse trascriptase-PCR, using Northern blots containing poly A+ mRNA, or any other direct or inderect RNA detection method. An increased or decreased expression of a given RNA in a diseased tissue compared to a normal tissue suggests that the transcript and/or the expressed protein has a role in the disease. Thus detection of a higher or lower level of mRNA corresponding to SEQ ID NO 1 or 3 relative to normal level is indicative of the presence of cancer in the patient.

mRNA expression levels in a sample can be determined by generation of a library of expressed sequence tags (ESTs) from the sample. The relative representation of ESTs in the library can be used to assess the relative representation of the gene transcript in the starting sample. The EST analysis of the test can then be compared to the EST analysis of a reference sample to determine the relative expression levels of the polynucleotide of interest.

Other mRNA analyses can be carried out using serial analysis of gene expression (SAGE) methodology (Velculescu et. Al. Science (1995) 270:484) , differential display methodology (For example, US 5,776,683) or hybridization analysis which relies on the specificity of nucleotide interactions.

Alternatively, the comparison could be made at the protein level. The protein sizes in the two tissues may be compared using antibodies to detect polypeptides in Western blots of protein extracts from the two tissues. Expression levels and subcellular localization may also be detected immunologically using antibodies to the corresponding protein. Further assay techniques that can be used to determine levels of a protein, such as a polypeptide of the present invention, in a sample derived from a host are well-known to those of skill in the art. A raised or decreased level of polypeptide expression in the diseased tissue compared with the same protein expression level in the normal tissue indicates that the expressed protein may be involved in the disease.

In the assays of the present invention, the diagnosis can be determined by detection of gene product expression levels encoded by at least one sequence set forth in SEQ ID NOS: 1 or 3. A comparison of the mRNA or protein levels in a diseased versus normal tissue may also be used to follow the progression or remission of a disease.

A large number of polynucleotide sequences in a sample can be assayed using polynucleotide arrays. These can be used to examine differential expression of genes and to determine gene function. For example, arrays of the polynucleotide sequences SEQ ID NO: 1 or 3 can be used to determine if any of the polynucleotides are differentially expressed between a normal and cancer cell. In one embodiment of the invention, an array of oligonucleotides probes comprising the SEQ ID NO: 1 or 3 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability (see for example: M.Chee et al., Science, Vol 274, pp 610-613 (1996)).

"Diagnosis" as used herein includes determination of a subject's susceptibility to a disease, determination as to whether a subject presently has the disease, and also the prognosis of a subject affected by the disease.

The present invention, further relates to a diagnostic kit for performing a diagnostic assay which comprises:

(a) a polynucleotide of the present invention, preferably the nucleotide sequence of SEQ ID NO: 1 or 3, or a fragment thereof ;
(b) a nucleotide sequence complementary to that of (a);
(c) a polypeptide of the present invention, preferably the polypeptide of SEQ ID NO: 2 or 4, or a fragment thereof; or
(d) an antibody to a polypeptide of the present invention, preferably to the polypeptide of SEQ ID NO:2 or 4.

The nucleotide sequences of the present invention are also valuable for chromosomal localisation. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined.

The polypeptides of the invention or their fragments or analogs thereof, or cells expressing them, can also be used as immunogens to produce antibodies immunospecific for polypeptides of the present invention. The term "immunospecific" means that the antibodies have substantially greater affinity for the polypeptides of the invention than their affinity for other related polypeptides in the prior art.

In a further aspect the invention provides an antibody immunospecific for a polypeptide according to the invention or an immunological fragment thereof as hereinbefore defined. Preferably the antibody is a monoclonal antibody

Antibodies generated against polypeptides of the present invention may be obtained by administering the polypeptides or epitope-bearing fragments, analogs or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor *et al*., Immunology Today (1983) 4:72) and the EBV-hybridoma technique (Cole *et al*., Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography.

The antibody of the invention may also be employed to prevent or treat cancer, particularly ovarian and colon cancer, autoimmune disease and related conditions.

Another aspect of the invention relates to a method for inducing or modulating an immunological response in a mammal which comprises inoculating the mammal with a polypeptide of the present invention, adequate to produce antibody and/or T cell immune response to protect or ameliorate the symptoms or progression of the disease. Yet another aspect of the invention relates to a method of inducing or modulating immunological response in a mammal which comprises, delivering a polypeptide of the present invention *via* a vector directing expression of the polynucleotide and coding for the polypeptide *in vivo* in order to induce such an immunological response to produce antibody to protect said animal from diseases.

It will be appreciated that the present invention therefore provides a method of treating abnormal conditions such as, for instance, cancer and autoimmune diseases, in particular, ovarian and colon cancer, related to either a presence of, an excess of, or an under-expression of, CASB616 polypeptide activity.

The present invention further provides for a method of screening compounds to identify those which stimulate or which inhibit the function of the CASB616 polypeptide. In general, agonists or antagonists may be employed for therapeutic and prophylactic purposes for such diseases as hereinbefore mentioned. Compounds may be identified from a variety of sources, for example, cells, cell-free preparations, chemical libraries, and natural products mixtures. Such agonists, antagonists or inhibitors so-identified may be natural or modified substrates, ligands, receptors, enzymes, etc., as the case may be, of the polypeptide; or may be structural or functional mimetics thereof (see Coligan *et al*., Current Protocols in Immunology 1(2): Chapter 5 (1991)). Screening methods will be known to those skilled in the art. Further screening methods may be found in for example D. Bennett *et al*., J Mol Recognition, 8:52-58 (1995): and K. Johanson *et al*., J Biol Chem, 270(16):9459-9471 (1995) and references therein.

Thus the invention provides a method for screening to identify compounds which stimulate or which inhibit the function of the polypeptide of the invention which comprises a method selected from the group consisting of:

(a) measuring the binding of a candidate compound to the polypeptide (or to the cells or membranes bearing the polypeptide) or a fusion protein thereof by means of a label directly or indirectly associated with the candidate compound;

(b) measuring the binding of a candidate compound to the polypeptide (or to the cells or membranes bearing the polypeptide) or a fusion protein thereof in the presense of a labeled competitor;

(c) testing whether the candidate compound results in a signal generated by activation or inhibition of the polypeptide, using detection systems appropriate to the cells or cell membranes bearing the polypeptide;

(d) mixing a candidate compound with a solution containing a polypeptide of claim 1, to form a mixture, measuring activity of the polypeptide in the mixture, and comparing the activity of the mixture to a standard; or

(e) detecting the effect of a candidate compound on the production of mRNA encoding said polypeptide and said polypeptide in cells, using for instance, an ELISA assay.

The polypeptide of the invention may be used to identify membrane bound or soluble receptors, if any, through standard receptor binding techniques known in the art. Well known screening methods may also be used to identify agonists and antagonists of the polypeptide of the invention which compete with the binding of the polypeptide of the invention to its receptors, if any.

Thus, in another aspect, the present invention relates to a screening kit for identifying agonists, antagonists, ligands, receptors, substrates, enzymes, etc. for polypeptides of the present invention; or compounds which decrease or enhance the production of such polypeptides, which comprises:

(a) a polypeptide of the present invention;
(b) a recombinant cell expressing a polypeptide of the present invention;
(c) a cell membrane expressing a polypeptide of the present invention; or
(d) antibody to a polypeptide of the present invention'

which polypeptide is preferably that of SEQ ID NO:2.

It will be readily appreciated by the skilled artisan that a polypeptide of the present invention may also be used in a method for the structure-based design of an agonist, antagonist or inhibitor of the polypeptide, by:

(a) determining in the first instance the three-dimensional structure of the polypeptide;
(b) deducing the three-dimensional structure for the likely reactive or binding site(s) of an agonist, antagonist or inhibitor;
(c) synthesing candidate compounds that are predicted to bind to or react with the deduced binding or reactive site; and
(d) testing whether the candidate compounds are indeed agonists, antagonists or inhibitors.

Gene therapy may also be employed to effect the endogenous production of CASB616 polypeptide by the relevant cells in the subject. For an overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996).

Vaccine preparation is generally described in Pharmaceutical Biotechnology, Vol.61 Vaccine Design - the subunit and adjuvant approach, edited by Powell and Newman, Plenurn Press, 1995. New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of protein in each vaccine dose is selected as an amount which induces an immunoprotective response without significant, adverse side effects in typical vaccinees. Such amount will vary depending upon which specific immunogen is employed. Generally, it is expected that each dose will comprise 1-1000μg of protein, preferably 2-100μg, most preferably 4-40μg. An optimal amount for a particular vaccine can be ascertained by standard studies involving observation of antibody titres and other responses in subjects. Following an initial vaccination, subjects may receive a boost in about 4 weeks.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA including single and double stranded regions.

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM *J. Applied Math., 48:* 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research *12(1):* 387 (1984)), BLASTP, BLASTN, and FASTA (Atschul, S.F. et al., J. Molec. Biol. *215*: 403-410 (1990). The BLAST X program is publicly available from NCBI and other sources (BLAST Manual, Altschul, S., *et al.*, NCBI NLM NIH Bethesda, MD 20894; Altschul, S., *et al.*, J. Mol. Biol. *215*: 403-410 (1990). The well known Smith Waterman algorithm may also be used to determine identity.

The preferred algorithm used is FASTA. The preferred parameters for polypeptide or polynuleotide sequence comparison using this algorithm include the following:

Gap Penalty: 12
Gap extension penalty: 4
Word size: 2, max 6

Preferred parameters for polypeptide sequence comparison with other methods include the following:

1) Algorithm: Needleman and Wunsch, J. Mol Biol. 48: 443-453 (1970)
Comparison matrix: BLOSSUM62 from Hentikoff and Hentikoff, Proc. Natl. Acad. Sci.
USA. 89:10915-10919 (1992)
Gap Penalty: 12
Gap Length Penalty: 4

A program useful with these parameters is publicly available as the "gap" program from Genetics Computer Group, Madison WI. The aforementioned parameters are the default parameters for polypeptide comparisons (along with no penalty for end gaps).

Preferred parameters for polynucleotide comparison include the following:

1) Algorithm: Needleman and Wunsch, J. Mol Biol. 48: 443-453 (1970)
Comparison matrix: matches = +10, mismatch = 0
Gap Penalty: 50
Gap Length Penalty: 3

A program useful with these parameters is publicly available as the "gap" program from Genetics Computer Group, Madison WI. The aforementioned parameters are the default parameters for polynucleotide comparisons.

By way of example, a polynucleotide sequence of the present invention may be identical to the reference sequence of SEQ ID NO:1, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO:1 by the numerical percent of the respective percent identity(divided by 100) and subtracting that product from said total number of nucleotides in SEQ ID NO:1, or:

$$n_n \leq x_n - (x_n \bullet y),$$

wherein $\mathbf{n}_n$ is the number of nucleotide alterations, $\mathbf{x}_n$ is the total number of nucleotides in SEQ ID NO:1, and **y** is, for instance, 0.70 for 70%, 0.80 for 80%, 0.85 for 85%, 0.90 for 90%, 0.95 for 95%,etc., and wherein any non-integer product of $\mathbf{x}_n$ and **y** is rounded down to the nearest integer prior to subtracting it from $\mathbf{x}_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

Similarly, a polypeptide sequence of the present invention may be identical to the reference sequence of SEQ ID NO:2, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the numerical percent of the respective percent identity(divided by 100) and then subtracting that product from said total number of amino acids in SEQ ID NO:2, or:

$$n_a \leq x_a - (x_a \bullet y),$$

wherein $\mathbf{n}_a$ is the number of amino acid alterations, $\mathbf{x}_a$ is the total number of amino acids in SEQ ID NO:2, and y is, for instance 0.70 for 70%, 0.80 for 80%, 0.85 for 85% etc., and wherein any non-integer product of $\mathbf{x}_a$ and **y** is rounded down to the nearest integer prior to subtracting it from $\mathbf{x}_a$.

"Homolog" is a generic term used in the art to indicate a polynucleotide or polypeptide sequence possessing a high degree of sequence relatedness to a subject sequence. Such relatedness may be quntified by determining the degree of identity and/or similarity between the sequences being compared as hereinbefore described. Falling within this generic term are the terms "ortholog", meaning a polynucleotide or polypeptide that is the functional equivalent of a polynucleotide or polypeptide in another species and "paralog" meaning a functionally similar sequence when considered within the same species.

## EXAMPLES

### Example 1 **Real-time RT-PCR analysis**

Real-time RT-PCR (U. Gibson. 1996. Genome Research: 6,996) is used to compare mRNA transcript abundance of the candidate antigen in matched tumour and normal colon tissues from multiple patients. In addition, mRNA levels of the candidate gene in a panel of normal tissues are evaluated by this approach.

Total RNA from normal and tumour colon is extracted from snap frozen biopsies using TriPure reagent (Boehringer). Total RNA from normal tissues is purchased from InVitrogen or is extracted from snap frozen biopsies using TriPure reagent. Poly-$A^+$ mRNA is purified from total RNA after DNAase treatment using oligo-dT magnetic beads (Dynal). Quantification of the mRNA is performed by spectrofluorimetry (VersaFluor, BioRad) using SybrII dye (Molecular Probes). Primers for real-time PCR amplification are designed with the Perkin-Elmer Primer Express software using default options for TaqMan amplification conditions.

Real-time reactions are assembled according to standard PCR protocols using 2 ng of purified mRNA for each reaction. SybrI dye (Molecular Probes) is added at a final dilution of 1/75000 for real-time detection. Amplification (40 cycles) and real-time detection is performed in a Perkin-Elmer Biosystems PE7700 system using conventional instrument settings. Ct values are calculated using the PE7700 Sequence Detector software. Two Ct values are obtained for each patient sample: the tumour Ct (CtT) and the matched normal colon Ct (CtN). Ct values obtained by real-time PCR are log-linearly related to the copy number of the target template. As the efficiency of PCR amplification under the prevailing experimental conditions is close to the theoretical amplification efficiency, $2^{(CtN-CtT)}$ is an estimate of the relative transcript levels in the two tissues (i.e. fold mRNA over-expression in tumor). Real-time PCR reactions are performed on biopsies from 17 patients. The level of mRNA over-expression is calculated as described for each patient. The average level of mRNA over-expression for the candidate antigen and the proportion of patients over-expressing the candidate antigen is then calculated from this data set.

For normal tissues, Ct values for the candidate antigen are compared to those of actin obtained with the same tissue sample.

| **Real-time PCR results in colon cancer/normal colon sample** | |
|---|---|
| **Summary** | |
| **Patients over-expressing CASB616 in colon tumours (%)** | **Average level of over-expression in colon tumours (fold)** |
| 17/17 (100%) | 17 |

**Details of the 3 experiments**

| | Experiment 1 (6 samples) | | | | | |
|---|---|---|---|---|---|---|
| | CtN (range) | CtN (mean) | CtT (range) | CtT (mean) | (CtN-CtT) (mean) | T over-expression (mean fold) |
| CASB616* Probe 1 | 30-34.5 | 32.3 | 28.5-31 | 29.8 | 2.5 | 5.7 |
| Probe 2 | | 26.3 | | 26 | 4 | 16 |
| Probe 3 | | 26.6 | | 27.1 | 2.7 | 6.5 |
| | **Experiment 2 (5 samples)** | | | | | |
| | CtN (range) | CtN (mean) | CtT (range) | CtT (mean) | (CtN-CtT) (mean) | T over-expression (mean fold) |
| CASB616 | 34-39 | 37.5 | 28-34.5 | 31 | 4.7 | 26 |
| | **Experiment 3 (6 samples)** | | | | | |
| | CtN (range) | CtN (mean) | CtT (range) | CtT (mean) | (CtN-CtT) (mean) | T over-expression (mean fold) |
| CASB616 | 38-40 | 39 | 29.5-35.5 | 32.5 | 7.7 | 207 |

* in the first experiment, 3 different probe pairs were used

**Real-time PCR results in normal tissues**

Ct values

| | *Bl* | *Bra* | *Sk* | *Ce* | *He* | *Ki* | *Li* | *Lu* | *Sp* | *Pl* | *Re* | *Te* |
|---|---|---|---|---|---|---|---|---|---|---|---|---|
| CASB616 | 31.5 | 29.5 | 20 | 32.5 | 34.5 | 31.5 | 30.5 | 31 | 30 | 29.5 | 28.5 | 31 |
| Actin | 16 | 17 | 18.5 | 17.5 | 17.5 | 17 | 15.5 | 16.5 | 30 | 16 | 16.5 | 16.5 |

| | *Ao* | *Ad Gl* | *Lu 2* | *Pr* | *Te 2* | *Oe* | *St* | *Co* | *Sk Mu* | *Ov* | *Fa Tu* | *Ce 2* |
|---|---|---|---|---|---|---|---|---|---|---|---|---|
| CASB616 | 24 | 30 | 21 | 29 | 22.5 | 33.5 | 30.5 | 22 | 30.5 | 24.5 | 25.5 | 28.5 |

| | | | | | | | | | | | | |
|---|---|---|---|---|---|---|---|---|---|---|---|---|
| Actin | 40 | 28.5 | 28.5 | 27 | 25.5 | 26 | 28 | 33.5 | 29 | 30.5 | 28.5 | 23.5 |

**Bl**: bladder; **Bra**: brain; **Sk:** skin; **Ce**: cervix; **He**: heart; **Ki**: kidney; **Li**: liver; **Lu**: lung; **Sp**: spleen; **Pl**: placenta; **Re**: rectum; **Te**: testis; **Ao**: aorta; **Ad Gl**: adrenal gland; **Pr**: prostate; **Oe**: esophagus; **St**: stomach; **Sk Mu**: skeletal muscle; **Ov**: ovary; **Fa Tu**: fallopian tube. Different samples of the same tissue have an additional numerical identifier.

**Conclusion**. CASB616 gene transcript is significantly over-expressed relative to normal colon in all primary colorectal tumors examined. Expression in the majority of normal tissues appears to be very low.

**Example 2.**

**DNA microarrays**

DNA micro-arrays are used to examine mRNA expression profiles of large collections of genes in multiple samples. This information is used to complement the data obtained by real-time PCR and provides an independent measure of gene expression levels in tumors and normal tissues.
Examples of current technologies for production of DNA micro-arrays include 1) The Affymetrix "GeneChip" arrays in which oligonucleotides are synthetized on the surface of the chip by solid phase chemical synthesis using a photolithographic process 2) DNA spotting technology in which small volumes of a DNA solution are robotically deposited and then immobilized onto the surface of a solid phase (e.g. glass). In both instances, the chips are hybridized with cDNA or cRNA which has been extracted from the tissue of interest (e.g. normal tissue, tumour etc...) and labeled with radioactivity or with a fluorescent reporter molecule. The labeled material is hybridized to the chip and the amount of probe bound to each sequence on the chip is determined using a specialized scanner. The experiment can be set-up with a single fluorescent reporter (or radioactivity) or, alternatively, can be performed using two fluorescent reporters. In this latter case, each of the two samples is labeled with one of the reporter molecules. The two labeled samples are then hybridized competitively to the sequences on the DNA chip. The ratio of the two fluorescent signals is determined for each sequence on the chip. This ratio is used to calculate the relative abundance of the transcript in the two samples.
Detailed protocols are available from a number of sources including "DNA Microarrays: A practical approach. Schena M. Oxford University Press 1999" and the World Wide Web (http://cmgm.stanford.edu/pbrown/protocols/index.html), http://arrayit.com/DNA-Microarray-Protocols/) and specialized distributors (e.g. Affymetrix).

**Example 3.**

**EST profiles**

A complementary approach to experimental antigen tissue expression characterization is to explore the human "Expressed Sequence Tags" (ESTs) database. ESTs are small fragments of cDNA made from a collection of mRNA extracted from a particular tissue or cell line. Such database currently provide a massive amount of ESTs ($10^6$) from several hundreds of cDNA tissue libraries, including tumoral tissues from various types and states of disease. By means of informatics tools, a comparison search of the CASB616 sequence is performed in order to have further insight into tissue expression.

**Results of the database search using CASB616 sequence**

| DbEST | ATG lib ID | Description | Category* |
|---|---|---|---|
| NCBI:580826 | 424 | fetal heart | F |
| NCBI:581388 | 424 | fetal heart | F |
| NCBI:584556 | 424 | fetal heart | F |
| NCBI:1160708 | 882 | NCI_CGAP_Co3 (12 pooled tumor colon) | TC |
| NCBI:1150180 | 895 | NCI_CGAP_Br2 (pooled breast tumor tissues) | TB |
| NCBI:1150189 | 895 | NCI_CGAP_Br2 (pooled breast tumor tissues) | TB |
| NCBI:1150229 | 895 | NCI_CGAP_Br2 (pooled breast tumor tissues) | TB |
| NCBI:1150370 | 895 | NCI_CGAP_Br2 (pooled breast tumor tissues) | TB |
| NCBI:1777532 | 843 | Fetus | F |
| NCBI:2127996 | 1541 | NCI_CGAP_Lu25 | Tlg |
| NCBI:2129674 | 1541 | NCI_CGAP_Lu25 | Tlg |
| NCBI:2129684 | 1541 | NCI_CGAP_Lu25 | Tlg |
| NCBI:2649403 | 1540 | NCI_CGAP_Co16 | TC |
| NCBI:3415191 | 1918 | Schneider fetal brain 00004 | F |
| NCBI:24019 | 2 | fetal brain | F |
| NCBI:1942 | 2 | fetal brain | F |
| NCBI:79438 | 226 | Human Placenta | N |
| NCBI:982827 | 695 | fetal kidney | F |

* F: fetal tissue libraries; TC: colon tumor libraries; TB: breast tumor libraries; Tlg: lung tumor libraries; N: normal tissue libraries.

**Example 4**

**Northern-Southern blot analysis**

Limited amounts of mixed tumour and matched normal colon cDNA are amplified by Advantage PCR (see above). Messenger RNA from multiple normal tissues is also amplified using the same procedure. The amplified cDNA (1 μg) is electrophoresed on a 1.2% agarose gel and transferred onto a nylon membrane. The membrane is hybridised (AlkPhos Direct System) with a probe prepared using a fragment of the candidate TAA cDNA. Northern-Southern analysis provides information on transcript size, presence of splice variants and transcript abundance in tumour and normal tissues.

**Example 5**

**Northern Blot Analysis**

Northern blots are produced according to standard protocols using 1 μg of poly A+ mRNA. Radioactive probes are prepared using the Ready-to-Go system (Pharmacia).

**Example 6 :**

**6.1 Expression and purification of tumour-specific antigens**

Expression in microbial hosts is used to produce the antigen of the invention for vaccine purposes and to produce protein fragments or whole protein for rapid purification and generation of antibodies needed for characterization of the naturally expressed protein by immunohistochemistry or for follow-up of purification.
Recombinant proteins may be expressed in two microbial hosts, *E. coli* and in yeast (such as Saccharomyces cerevisiae or Pichia pastoris).Pichia. This allows the selection of the expression system with the best features for this particular antigen production. In general, the recombinant antigen will be expressed in *E. coli* and the reagent protein expressed in yeast.
The expression strategy first involves the design of the primary structure of the recombinant antigen. In general an expression fusion partner (EFP) is placed at the N terminal extremity to improve levels of expression that could also include a region useful for modulating the immunogenic properties of the antigen, an immune fusion partner (IFP). In addition, an affinity fusion partner (AFP ) useful for facilitating further purification is included at the C-terminal end.

When the recombinant strains are available, the recombinant product is characterized by the evaluation of the level of expression and the prediction of further solubility of the protein by analysis of the behavior in the crude extract.
After growth on appropriate culture medium and induction of the recombinant protein expression, total extracts are analyzed by SDS-PAGE. The recombinant proteins are visualized in stained gels and identified by Western blot analysis using specific antibodies.
A comparative evaluation of the different versions of the expressed antigen will allow the selection of the most promising candidate that is to be used for further purification and immunological evaluation.
The purification work follows a classical approach based on the presence of an His affinity tail in the recombinant protein. In a typical experiment the disrupted cells are filtered and the acellular extracts loaded onto an Ion Metal Affinity Chromatography (IMAC; $Ni^{++}$NTA from Qiagen) that will specifically retain the recombinant protein.
The retained proteins are eluted by 0-500 mM Imidazole gradient (possibly in presence of a detergent) in a phosphate buffer. This step is optimally followed by an Anion Exchange resin step and a Size Exclusion chromatography step depending on the success of the Imac step and the nature of the contaminants.

### 6.2 Antibody production and immunohistochemistry

Small amounts of relatively purified protein can be used to generate immunological tools in order to

a) detect the expression by immunohistochemistry in normal or cancer tissue sections;
b) detect the expression, and to follow the protein during the purification process (ELISA/ Western Blot); or
c) characterise/ quantify the purified protein (ELISA).

#### 6.2.1 Polyclonal antibodies:

***Immunization***

2- 3 Rabbits are immunized , intramuscularly (I.M.) , 3 times at 3 weeks intervals with 100µg of protein, formulated in the adjuvant 3D-MPL/QS21 . 3 weeks after each immunisation a blood sample is taken and the antibody titer estimated in the serum by ELISA using the protein as coating antigen following a standard protocol.

***ELISA***

96 well microplates (maxisorb Nunc) are coated with 5µg of protein overnight at 4°C. After 1hour saturation at 37°C with PBS NCS 1%, serial dilution of the rabbit sera is added for 1H 30 at 37°C (starting at 1/10). After 3 washings in PBS Tween, anti rabbit biotinylated anti serum (Amersham ) is added (1/5000). Plates are washed and peroxydase coupled streptavidin (1/5000) is added for 30 min at 37°C. After washing, 50µl TMB (BioRad) is added for 7 min and the reaction then stopped with $H_2SO_4$ 0.2M. The OD can be measured at 450 nm and midpoint dilutions calculated by SoftmaxPro.

#### 6.2.2 Monoclonal antibodies:

***Immunization***

5 BALB/c mice are immunized 3 times at 3 week intervals with 5 µg of purified protein. Bleedings are performed 14 days post II and 1 week post 3. The sera is tested by Elisa on purified protein used as coated antigen. Based on these results (midpoint dilution > 10000 ) one mouse is selected for fusion

***Fusion/ HATselection***

Spleen cells are fused with the SP2/0 myeloma according to a standard protocol using PEG 40% and DMSO 5%. Cells are then seeded in 96 well plates 2.5 x$10^4$ - $10^5$ cells/well and resistant clones will be selected in HAT medium. The supernatant of these hybridomas will be tested for their content in specific antibodies and when positive, will be submitted to 2 cycles of limited dilution . After 2 rounds of screening, 3 hybridomas will be chosen for ascitis production.

### 6.2.3 Immunohistochemistry

When antibodies are available, immuno staining is performed on normal or cancer tissue sections, in order to determine :

- the level of expression of the antigen of the invention in cancer relative to normal tissue or
- the proportion of cancer of a certain type expressing the antigen
- if other cancer types also express the antigen
- the proportion of cells expressing the antigen in a cancer tissue

*Tissue sample preparation*

After dissection, the tissue sample is mounted on a cork disk in OCT compound and rapidly frozen in isopentane previously super cooled in liquid nitrogen (-160°C). The block will then be conserved at -70°C until use. 7-10µm sections will be realized in a cryostat chamber (-20, -30°C).

*Staining*

Tissue sections are dried for 5 min at room Temperature (RT), fixed in acetone for 10min at RT,dried again, and saturated with PBS 0.5% BSA 5% serum. After 30 min at RT either a direct or indirect staining is performed using antigen specific antibodies. A direct staining leads to a better specificity but a less intense staining whilst an indirect staining leads to a more intense but less specific staining.

### 6.4 Analysis of human cellular immune responses to the antigen of the invention

The immunological relevance of the antigen of the invention can be assessed by *in vitro* priming of human T cells. All T cell lymphocyte lines and dendritic cells are derived from PBMCs (peripheral blood mononuclear cells) of healthy donors (preferred HLA-A2 subtype). An HLA-A2.1/$K^b$ transgenic mice is also used for screening of HLA-A2.1 peptides.

Newly discovered antigen-specific CD8+ T cell lines are raised and maintained by weekly in vitro stimulation. The lytic activity and the γ-IFN production of the CD8 lines in response to the antigen or antigen derived-peptides is tested using standard procedures.

Two strategies to raise the CD8+ T cell lines are used: a peptide-based approach and a whole gene-based approach. Both approaches require the full-length cDNA of the newly discovered antigen in the correct reading frame to be either cloned in an appropriate delivery system or to be used to predict the sequence of HLA binding peptides.

***Peptide-based approach***

The HLA-A2 binding peptide sequences are predicted by the Parker's algorithm. Peptides are then screened in the HLA-A2.1/$K^b$ transgenic mice model (Vitiello et al.). The sequence used to perform the prediction is EPHB2v, as it is identical to EPHB2 with an additional C-terminal sequence extension.

| Predicted epitopes binding the HLA_A0201 allele : | | | |
|---|---|---|---|
| SEQ ID NO | Start Position | Subsequence Residue Listing | Score* |
| 5 | 948 | MMMEDILRV | 2979.496 |
| 6 | 11 | LLLPLLAAV | 1006.209 |
| 7 | 387 | GLTEPRIYI | 235.260 |
| 8 | 712 | RQNDGQFTV | 167.692 |
| 9 | 22 | TLMDSTTAT | 113.047 |
| 10 | 641 | KLPGkREIFV | 1338.876 |

* Estimate of Half Time of Disassociation of a Molecule Containing This Subsequence

(continued)

| Predicted epitopes binding the HLA_A0201 allele : | | | |
|---|---|---|---|
| SEQ ID NO | Start Position | Subsequence Residue Listing | Score* |
| 11 | 10 | LLLLpLLAAV | 1006.209 |
| 12 | 947 | QMMMeDILRV | 427.474 |
| 13 | 810 | WSYGiVMWEV | 115.285 |
| 14 | 554 | FLIAvVVIAI | 110.379 |

* Estimate of Half Time of Disassociation of a Molecule Containing This Subsequence

Briefly, transgenic mice are immunized with adjuvanted HLA-A2 peptides, those unable to induce a CD8 response (as defined by an efficient lysis of peptide-pulsed autologous spleen cells) will be further analyzed in the human system.
Human dendritic cells (cultured according to Romani et al.) will be pulsed with peptides and used to stimulated CD8-sorted T cells (by Facs). After several weekly stimulations, the CD8 lines will be first tested on peptide-pulsed autologous BLCL (EBV-B transformed cell lines). To verify the proper *in vivo* processing of the peptide, the CD8 lines will be tested on cDNA-transfected tumour cells (HLA-A2 transfected LnCaP, Skov3 or CAMA tumour cells).

***Whole gene-based approach***

CD8+ T cell lines will be primed and stimulated with either gene-gun transfected dendritic cells, retrovirally transduced B7.1-transfected fibroblastes, recombinant pox virus (Kim et al.) or adenovirus (Butterfield et al.) infected dentridic cells. Virus infected cells are very efficient to present antigenic peptides since the antigen is expressed at high level but can only be used once to avoid the over-growth of viral T cells lines.
After alternated stimulations, the CD8 lines are tested on cDNA-transfected tumour cells as indicated above. Peptide specificity and identity is determined to confirm the immunological validation.
**References**

Vitiello et al. (L. Sherman), J. Exp. Med., J. Exp. Med, 1991, 173:1007-1015.
Romani et al., J. Exp. Med., 1994, 180:83-93.
Kim et al., J. Immunother., 1997, 20:276-286.
Butterfield et al., J. Immunol., 1998, 161:5607-5613.

All publications, including but not limited to patents and patent applications, cited in this specification are herein incorporated by reference as if each individual publication were specifically and individually indicated to be incorporated by reference herein as though fully set forth.

# SEQUENCE INFORMATION

## SEQ ID NO:1

```
   1 aacaaaagct ggagctccac cgcggtggcg gccgctctag cccctggttc ggcccacctc
  61 tgaaggttcc agaatcgata gtgaattcgt ggggaagcgc agccatggct ctgcggaggc
 121 tgggggccgc gctgctgctg ctgccgctgc tcgccgccgt ggaagaaacg ctaatggact
 181 ccactacagc gactgctgag ctgggctgga tggtgcatcc tccatcaggg tgggaagagg
 241 tgagtggcta cgatgagaac atgaacacga tccgcacgta ccaggtgtgc aacgtgtttg
 301 agtcaagcca gaacaactgg ctacggacca agtttatccg gcgccgtggc gcccaccgca
 361 tccacgtgga gatgaagttt tcggtgcgtg actgcagcag catccccagc gtgcctggct
 421 cctgcaagga gaccttcaac ctctattact atgaggctga ctttgactcg gccaccaaga
 481 ccttccccaa ctggatggag aatccatggg tgaaggtgga taccattgca gccgacgaga
 541 gcttctccca ggtggacctg ggtggccgcg tcatgaaaat caacaccgag gtgcggagct
 601 tcggacctgt gtcccgcagc ggcttctacc tggccttcca ggactatggc ggctgcatgt
 661 ccctcatcgc cgtgcgtgtc ttctaccgca agtgcccccg catcatccag aatggcgcca
 721 tcttccagga aaccctgtcg gggggctgaga gcacatcgct ggtggctgcc cggggcagct
 781 gcatcgccaa tgcggaagag gtggatgtac ccatcaagct ctactgtaac ggggacggcg
 841 agtggctggt gcccatcggg cgctgcatgt gcaaagcagg cttcgaggcc gttgagaatg
 901 gcaccgtctg ccgaggttgt ccatctggga ctttcaaggc caaccaaggg gatgaggcct
 961 gtacccactg tcccatcaac agccggacca cttctgaagg ggccaccaac tgtgtctgcc
1021 gcaatggcta ctacagagca gacctggacc ccctggacat gccctgcaca accatcccct
1081 ccgcgcccca ggctgtgatt tccagtgtca atgagacctc cctcatgctg gagtggaccc
1141 ctccccgcga ctccggaggc cgagaggacc tcgtctacaa catcatctgc aagagctgtg
1201 gctcgggccg gggtgcctgc acccgctgcg gggacaatgt acagtacgca ccacgccagc
1261 taggcctgac cgagccacgc atttacatca gtgacctgct ggcccacacc cagtacacct
1321 tcgagatcca ggctgtgaac ggcgttactg accagagccc cttctcgcct cagttcgcct
1381 ctgtgaacat caccaccaac caggcagctc catcggcagt gtccatcatg catcaggtga
1441 gccgcaccgt ggacagcatt accctgtcgt ggtcccagcc agaccagccc aatggcgtga
1501 tcctggacta tgagctgcag tactatgaga aggagctcag tgagtacaac gccacagcca
1561 taaaaagccc caccaacacg gtcaccgtgc agggcctcaa agccggcgcc atctatgtct
1621 tccaggtgcg ggcacgcacc gtggcaggct acgggcgcta cagcggcaag atgtacttcc
1681 agaccatgac agaagccgag taccagacaa gcatccagga gaagttgcca ctcatcatcg
1741 gctcctcggc cgctggcctg gtcttcctca ttgctgtggt tgtcatcgcc atcgtgtgta
1801 acagaagacg ggggtttgag cgtgctgact cggagtacac ggacaagctg caacactaca
1861 ccagtggcca catgacccca ggcatgaaga tctacatcga tcctttcacc tacgaggacc
1921 ccaacgaggc agtgcgggag tttgccaagg aaattgacat ctcctgtgtc aaaattgagc
1981 aggtgatcgg agcaggggag tttggcgagg tctgcagtgg ccacctgaag ctgccaggca
2041 agagagagat ctttgtggcc atcaagacgc tcaagtcggg ctacacggag aagcagcgcc
2101 gggacttcct gagcgaagcc tccatcatgg gccagttcga ccatcccaac gtcatccacc
2161 tggagggtgt cgtgaccaag agcacacctg tgatgatcat caccgagttc atggagaatg
2221 gctccctgga ctcctttctc cggcaaaacg atgggcagtt cacagtcatc cagctggtgg
2281 gcatgcttcg gggcatcgca gctggcatga agtacctggc agacatgaac tatgttcacc
2341 gtgacctggc tgcccgcaac atcctcgtca acagcaacct ggtctgcaag gtgtcggact
2401 ttgggctctc acgctttcta gaggacgata cctcagaccc cacctacacc agtgccctgg
2461 gcggaaagat ccccatccgc tggacagccc cggaagccat ccagtaccgg aagttcacct
2521 cggccagtga tgtgtggagc tacggcattg tcatgtggga ggtgatgtcc tatggggagc
2581 ggccctactg ggacatgacc aaccaggatg taatcaatgc cattgagcag gactatcggc
2641 tgccaccgcc catggactgc ccgagcgccc tgcaccaact catgctggac tgttggcaga
2701 aggaccgcaa ccaccggccc aagttcggcc aaattgtcaa cacgctagac aagatgatcc
2761 gcaatcccaa cagcctcaaa gccatggcgc ccctctcctc tggcatcaac ctgccgctgc
2821 tggaccgcac gatccccgac tacaccagct ttaacacggt ggacgagtgg ctggaggcca
2881 tcaagatggg gcagtacaag gagagcttcg ccaatgccgg cttcacctcc tttgacgtcg
2941 tgtctcagat gatgatggag gacattctcc gggttgggct cactttggct ggccaccaga
3001 aaaaaatcct gaacagtatc caggtgatgc gggcgcagat gaaccagatt cagtctgtgg
3061 aggtttgaca ttcacctgcc tcggctcacc tcttcctcca agccccgccc cctctgcccc
3121 acgtgccggc cctcctggtg ctctatccac tgcagggcca gccactcgcc aggaggccac
3181 gggccacggg aagaaccaag cggtgccagc cacgagacgt caccaagaaa acatgcaact
3241 caaacgacgg aaaaaaaaag ggaatgggaa aaaagaaaac agatcctggg agggggcggg
3301 aaatacaagg aatatttttt aaagaggatt ctcataagga aagcaatgac tgttcttgcg
3361 ggggataaaa aagggcttgg gagattcatg cgatgtgtcc aatcggagac aaaagcagtt
3421 tctctccaac tccctctggg aaggtgacct ggccagagcc aagaaacact ttcagaaaaa
```

```
3481 caaatgtgaa ggggagagac aggggccgcc cttggctcct gtccctgctg ctcctctagg
3541 cctcactcaa caaccaagcg cctggaggac gggacagatg gacagacagc caccctgaga
3601 acccctctgg gaaaatctat tcctgccacc actgggcaaa cagaagaatt tttctgtctt
3661 tggagagtat tttagaaact ccaatgaaag acactgtttc tcctgttggc tcacagggct
3721 gaaaggggct tttgtcctcc tgggtcaggg agaacgcggg gaccccag//
```

## SEQ ID No. 2

```
  1 malrrlgaal lllpllaave etlmdsttat aelgwmvhpp sgweevsgyd enmntirtyq
 61 vcnvfessqn nwlrtkfirr rgahrihvem kfsvrdcssi psvpgscket fnlyyyeadf
121 dsatktfpnw menpwvkvdt iaadesfsqv dlggrvmkin tevrsfgpvs rsgfylafqd
181 yggcmsliav rvfyrkcpri iqngaifqet lsgaestslv aargsciana eevdvpikly
241 cngdgewlvp igrcmckagf eavengtvcr gcpsgtfkan qgdeacthcp insrttsega
301 tncvcrngyy radldpldmp cttipsapqa vissvnetsl mlewtpprds ggredlvyni
361 ickscgsgrg actrcgdnvq yaprqlglte priyisdlla htqytfeiqa vngvtdqspf
421 spqfasvnit tnqaapsavs imhqvsrtvd sitlswsqpd qpngvildye lqyyekelse
481 ynataikspt ntvtvqglka gaiyvfqvra rtvagygrys gkmyfqtmte aeyqtsiqek
541 lpliigssaa glvfliavvv iaivcnrrgf eradseytdk lqhytsghmt pgmkiyidpf
601 tyedpneavr efakeidisc vkieqvigag efgevcsghl klpgkreifv aiktlksgyt
661 ekqrrdflse asimgqfdhp nvihlegvvt kstpvmiite fmengsldsf lrqndgqftv
721 iqlvgmlrgi aagmkyladm nyvhrdlaar nilvnsnlvc kvsdfglsrf leddtsdpty
781 tsalggkipi rwtapeaiqy rkftsasdvw sygivmwevm sygerpywdm tnqdvinaie
841 qdyrlpppmd cpsalhqlml dcwqkdrnhr pkfgqivntl dkmirnpnsl kamaplssgi
901 nlplldrtip dytsfntvde wleaikmgqy kesfanagft sfdvvsqmmm edilrlgvtl
961 aghqkkilns iqvmraqmnq iqsvev
```

## SEQ ID No. 3

```
   1 gaattccgcc ccgggaagcg cagccatggc tctgcggagg ctgggggccg cgctgctgct
  61 gctgccgctg ctcgccgccg tggaagaaac gctaatggac tccactacag cgactgctga
 121 gctgggctgg atggtgcatc ctccatcagg gtgggaagag gtgagtggct acgatgagaa
 181 catgaacacg atccgcacgt accaggtgtg caacgtgttt gagtcaagcc agaacaactg
 241 gctacggacc aagtttatcc ggcgccgtgg cgcccaccgc atccacgtgg agatgaagtt
 301 ttcggtgcgt gactgcagca gcatccccag cgtgcctggc tcctgcaagg agaccttcaa
 361 cctctattac tatgaggctg actttgactc ggccaccaag accttcccca actggatgga
 421 gaatccatgg gtgaaggtgg ataccattgc agccgacgag agcttctccc aggtggacct
 481 gggtggccgc gtcatgaaaa tcaacaccga ggtgcggagc ttcggacctg tgtcccgcag
 541 cggcttctac ctggccttcc aggactatgg cggctgcatg tccctcatcg ccgtgcgtgt
 601 cttctaccgc aagtgccccc gcatcatcca gaatggcgcc atcttccagg aaaccctgtc
 661 gggggctgag agcacatcgc tggtggctgc ccggggcagc tgcatcgcca atgcggaaga
 721 ggtggatgta cccatcaagc tctactgtaa cggggacggc gagtggctgg tgcccatcgg
 781 gcgctgcatg tgcaaagcag gcttcgaggc cgttgagaat ggcaccgtct gccgaggttg
 841 tccatctggg actttcaagg ccaaccaagg ggatgaggcc tgtacccact gtcccatcaa
 901 cagccggacc acttctgaag ggccaccaa ctgtgtctgc cgcaatggct actacagagc
 961 agacctggac cccctggaca tgccctgcac aaccatcccc tccgcgcccc aggctgtgat
1021 ttccagtgtc aatgagacct ccctcatgct ggagtggacc cctccccgcg actccggagg
1081 ccgagaggac ctcgtctaca acatcatctg caagagctgt ggctcgggcc ggggtgcctg
1141 cacccgctgc ggggacaatg tacagtacgc accacgccag ctaggcctga ccgagccacg
1201 catttacatc agtgacctgc tggcccacac ccagtacacc ttcgagatcc aggctgtgaa
1261 cggcgttact gaccagagcc ccttctcgcc tcagttcgcc tctgtgaaca tcaccaccaa
1321 ccaggcagct ccatcggcag tgtccatcat gcatcaggtg agccgcaccg tggacagcat
1381 taccctgtcg tggtcccagc cagaccagcc caatggcgtg atcctggact atgagctgca
1441 gtactatgag aaggagctca gtgagtacaa cgccacagcc ataaaaagcc ccaccaacac
1501 ggtcaccgtg cagggcctca aagccggcgc catctatgtc ttccaggtgc gggcacgcac
1561 cgtggcaggc tacgggcgct acagcggcaa gatgtacttc cagaccatga cagaagccga
1621 gtaccagaca agcatccagg agaagttgcc actcatcatc ggctcctcgg ccgctggcct
1681 ggtcttcctc attgctgtgg ttgtcatcgc catcgtgtgt aacagacggg ggtttgagcg
1741 tgctgactcg gagtacacgg acaagctgca acactacacc agtggccaca tgaccccagg
1801 catgaagatc tacatcgatc ctttcaccta cgaggacccc aacgaggcag tgcgggagtt
```

```
1861 tgccaaggaa attgacatct cctgtgtcaa aattgagcag gtgatcggag caggggagtt
1921 tggcgaggtc tgcagtggcc acctgaagct gccaggcaag agagagatct ttgtggccat
1981 caagacgctc aagtcgggct acacggagaa gcagcgccgg gacttcctga gcgaagcctc
2041 catcatgggc cagttcgacc atcccaacgt catccacctg gagggtgtcg tgaccaagag
2101 cacacctgtg atgatcatca ccgagttcat ggagaatggc tccctggact cctttctccg
2161 gcaaaacgat gggcagttca cagtcatcca gctggtgggc atgcttcggg gcatcgcagc
2221 tggcatgaag tacctggcag acatgaacta tgttcaccgt gacctggctg cccgcaacat
2281 cctcgtcaac agcaacctgg tctgcaaggt gtcggacttt gggctctcac gctttctaga
2341 ggacgatacc tcagacccca cctacaccag tgccctgggc ggaaagatcc ccatccgctg
2401 gacagccccg gaagccatcc agtaccggaa gttcacctcg gccagtgatg tgtggagcta
2461 cggcattgtc atgtgggagg tgatgtccta tggggagcgg ccctactggg acatgaccaa
2521 ccaggatgta atcaatgcca ttgagcagga ctatcggctg ccaccgccca tggactgccc
2581 gagcgccctg caccaactca tgctggactg ttggcagaag gaccgcaacc accggcccaa
2641 gttcggccaa attgtcaaca cgctagacaa gatgatccgc aatcccaaca gcctcaaagc
2701 catggcgccc ctctcctctg gcatcaacct gccgctgctg gaccgcacga tccccgacta
2761 caccagcttt aacacggtgg acgagtggct ggaggccatc aagatggggc agtacaagga
2821 gagcttcgcc aatgccggct tcacctcctt tgacgtcgtg tctcagatga tgatggagga
2881 cattctccgg gttggggtca ctttggctgg ccaccagaaa aaaatcctga acagtatcca
2941 ggtgatgcgg gcgcagatga accagattca gtctgtggag ggccagccac tcgccaggag
3001 gccacgggcc acgggaagaa ccaagcggtg ccagccacga gacgtcacca agaaaacatg
3061 caactcaaac gacggaaaaa aaaagggaat gggaaaaaag aaaacagatc ctgggagggg
3121 gcgggaaata caaggaatat tttttaaaga ggattctcat aaggaaagca atgactgttc
3181 ttgcggggga taaaaaaggg cttgggagat tcatgcgatg tgtccaatcg gagacaaaag
3241 cagtttctct ccaactccct ctgggaaggt gacctggcca gagccaagaa acactttcag
3301 aaaaacaaat gtgaagggga gagacagggg ccacccttgg ctcctgtccc tgctgctcct
3361 ctaggcctca ctcaacaacc aagcgcctgg aggacgggac agatggacag acagccaccc
3421 tgagaacccc tctgggaaaa tctattcctg ccaccactgg gcaaacagaa gaatttttct
3481 gtctttggag agtattttag aaactccaat gaaagacact gtttctcctg ttggctcaca
3541 gggctgaaag gggcttttgt cctcctgggt cagggagaac gcggggaccc cagaaaggtc
3601 agccttcctg aggatgggca acccccaggt ctgcagctcc aggtacatat cacgcgcaca
3661 gcctggcagc ctggccctcc tggtgcccac tcccgccagc ccctgcctcg aggactgata
3721 ctgcagtgac tgccgtcagc tccgactgcc gctgagaagg gttgatcctg catctgggtt
3781 tgtttacagc aattcctgga ctcgggggta ttttggtcac agggtggttt tggtttaggg
3841 ggtttgtttg ttgggttgtt ttttgttttt tggttttttt taatgacaat gaagtgacac
3901 tttgacattt ccaaaaaaaa aaaaaaaaaa aaaaaaaaaa aaaaaaaaa//
```

**SEQ ID No. 4**

```
   1 malrrlgaal lllpllaave etlmdsttat aelgwmvhpp sgweevsgyd enmntirtyq
  61 vcnvfessqn nwlrtkfirr rgahrihvem kfsvrdcssi psvpgscket fnlyyyeadf
 121 dsatktfpnw menpwvkvdt iaadesfsqv dlggrvmkin tevrsfgpvs rsgfylafqd
 181 yggcmsliav rvfyrkcpri iqngaifqet lsgaestslv aargsciana eevdvpikly
 241 cngdgewlvp igrcmckagf eavengtvcr gcpsgtfkan qgdeacthcp insrttsega
 301 tncvcrngyy radldpldmp cttipsapqa vissvnetsl mlewtpprds ggredlvyni
 361 ickscgsgrg actrcgdnvq yaprqlglte priyisdlla htqytfeiqa vngvtdqspf
 421 spqfasvnit tnqaapsavs imhqvsrtvd sitlswsqpd qpngvildye lqyyekelse
 481 ynataikspt ntvtvqglka gaiyvfqvra rtvagygrys gkmyfqtmte aeyqtsiqek
 541 lpliigssaa glvfliavvv iaivcnrrgf eradseytdk lqhytsghmt pgmkiyidpf
 601 tyedpneavr efakeidisc vkieqvigag efgevcsghl klpgkreifv aiktlksgyt
 661 ekqrrdflse asimgqfdhp nvihlegvvt kstpvmiite fmengsldsf lrqndgqftv
 721 iqlvgmlrgi aagmkyladm nyvhrdlaar nilvnsnlvc kvsdfglsrf leddtsdpty
 781 tsalggkipi rwtapeaiqy rkftsasdvw sygivmwevm sygerpywdm tnqdvinaie
 841 qdyrlpppmd cpsalhqlml dcwqkdrnhr pkfgqivntl dkmirnpnsl kamaplssgi
 901 nlplldrtip dytsfntvde wleaikmgqy kesfanagft sfdvvsqmmm edilrvgvtl
 961 aghqkkilns iqvmraqmnq iqsvegqpla rrpratgrtk rcqprdvtkk tcnsndgkkk
1021 gmgkkktdpg rgreiqgiff kedshkesnd cscgg
```

**SEQ ID NO:5**
MMMEDILRV

**SEQ ID NO:6**

LLLPLLAAV

**SEQ ID NO:7**
GLTEPRIYI

**SEQ ID NO:8**
RQNDGQFTV

**SEQ ID NO:9**
TLMDSTTAT

**SEQ ID NO:10**
KLPGkREIFV

**SEQ ID NO:11**
LLLLpLLAAV

**SEQ ID NO:12**
QMMMeDILRV
**SEQ ID NO:13**

WSYGiVMWEV

**SEQ ID NO:14**
FLIAvVVIAI

## Claims

**1.** A vaccine composition comprising an effective amount of a polypeptide which polypeptide comprises an amino acid sequence which has at least 85% identity to the amino acid sequence of SEQ ID NO: 2 or 4 or to an immunogenic fragment thereof, together with a pharmaceutically acceptable carrier.

**2.** A vaccine composition according to claim 1 wherein the amino acid sequence has at least 95% identity to the amino acid sequence of SEQ ID NO: 2 or 4 or to an immunogenic fragment thereof.

**3.** A vaccine composition comprising an effective amount of a polynucleotide which polynucleotide comprises a nucleotide sequence which has at least 85% identity to the nucleotide sequence of SEQ ID NO: 1, 3 or to a fragment thereof which encodes an immunogenic polypeptide, together with a pharmaceutically acceptable carrier.

**4.** A vaccine composition comprising an effective amount of antigen presenting cells, modified by in vitro loading with a polypeptide of SEQ ID NO:2 or 4, or genetically modified in vitro to express a polypeptide of SEQ ID NO:2 or 4 and a pharmaceutically effective carrier.

**5.** A vaccine as claimed in any one of claims 1 to 4 which additionally comprises a TH-1 inducing adjuvant.

**6.** A vaccine as claimed in claim 5 in which the TH-1 inducing adjuvant is selected from the group of adjuvants comprising: 3D-MPL, QS21, a mixture of QS21 and cholesterol, and a CpG oligonucleotide.

**7.** An antibody immunospecific for the polypeptide or immunological fragment as defined in any one of claims 1 to 6.

**8.** A method for screening to identify compounds which stimulate or which inhibit the function of the polypeptide of SEQ ID NO: 2 or 4 which comprises a method selected from the group consisting of:

(a) measuring the binding of a candidate compound to the said polypeptide (or to the cells or membranes bearing the polypeptide) or a fusion protein thereof by means of a label directly or indirectly associated with the candidate compound;
(b) measuring the binding of a candidate compound to the said polypeptide (or to the cells or membranes bearing the polypeptide) or a fusion protein thereof in the presense of a labeled competitior;
(c) testing whether the candidate compound results in a signal generated by activation or inhibition of the said polypeptide, using detection systems appropriate to the cells or cell membranes bearing the polypeptide;
(d) mixing a candidate compound with a solution containing a polypeptide of SEQ ID NO: 2 or 4, to form a mixture, measuring activity of the polypeptide in the mixture, and comparing the activity of the mixture to a standard; or
(e) detecting the effect of a candidate compound on the production of mRNA encoding said polypeptide and said polypeptide in cells, using for instance, an ELISA assay.

**9.** A method for the treatment of a subject by immunoprophylaxis or therapy comprising *in vitro* induction of immune responses to a molecule of any one of SEQ ID NOS: 1 - 4, using *in vitro* incubation of the polypeptide of SEQ ID NO: 2 or 4 or the polynucleotide of SEQ ID NO: 1 or 3 with cells from the immune system of a mammal, and reinfusing these activated immune cells to the mammal for the treatment of disease.

**10.** A method as claimed in claim 9 wherein the treatment is for ovarian or colon cancer.

**11.** An agonist or antagonist to the polypeptide of SEQ ID NO: 2 or 4.

**12.** A compound which is:

(a) an agonist or antagonist to the polypeptide of SEQ ID NO: 2 or 4;
(b) isolated polynucleotide of SEQ ID NO: 1 or 3; or
(c) a nucleic acid molecule that modulates the expression of the nucleotide sequence encoding the polypeptide of any one of SEQ ID NO: 2 or 4;

for use in therapy.

**13.** A process for diagnosing a disease or a susceptibility to a disease in a subject related to expression or activity of a polypeptide of SEQ ID NO: 2 or 4 in a subject comprising analyzing for the presence or amount of said polypeptide in a sample derived from said subject.

**14.** A process for diagnosing a disease or a susceptibility to a disease in a subject related to expression or activity of a polynucleotide of SEQ ID NO: 1 or 3 in a subject comprising analyzing for the presence or amount of said polynucleotide in a sample derived from said subj ect.

**15.** A process for diagnosing the presence of colon cancer or a susceptibility to colon cancer in a subject related to expression or activity of a polypeptide of SEQ ID NO: 2 or 4 in a subject comprising analyzing for the presence or amount of said polypeptide in a sample derived from said subject.

**16.** A process for diagnosing the presence of colon cancer or a susceptibility to colon cancer in a subject related to expression or activity of a polynucleotide of SEQ ID NO: 1 or 3 in a subject comprising analyzing for the presence or amount of said polynucleotide in a sample derived from said subject.

SEQUENCE LISTING

<110> SmithKline Beecham Biologicals S.A.

<120> Novel Uses

<130> BC45244

<160> 14

<170> FastSEQ for Windows Version 3.0

<210> 1
<211> 3768
<212> DNA
<213> Homo sapiens

<400> 1

```
aacaaaagct ggagctccac cgcggtggcg gccgctctag cccctggttc ggcccacctc      60
tgaaggttcc agaatcgata gtgaattcgt ggggaagcgc agccatggct ctgcggaggc     120
tgggggccgc gctgctgctg ctgccgctgc tcgccgccgt ggaagaaacg ctaatggact     180
ccactacagc gactgctgag ctgggctgga tggtgcatcc tccatcaggg tgggaagagg     240
tgagtggcta cgatgagaac atgaacacga tccgcacgta ccaggtgtgc aacgtgtttg     300
agtcaagcca gaacaactgg ctacggacca agtttatccg gcgccgtggc gcccaccgca     360
tccacgtgga gatgaagttt tcggtgcgtg actgcagcag catccccagc gtgcctggct     420
cctgcaagga gaccttcaac ctctattact atgaggctga ctttgactcg gccaccaaga     480
ccttccccaa ctggatggag aatccatggg tgaaggtgga taccattgca gccgacgaga     540
gcttctccca ggtggacctg ggtggccgcg tcatgaaaat caacaccgag gtgcggagct     600
tcggacctgt gtcccgcagc ggcttctacc tggccttcca ggactatggc ggctgcatgt     660
ccctcatcgc cgtgcgtgtc ttctaccgca agtgcccccg catcatccag aatggcgcca     720
tcttccagga aaccctgtcg gggctgaga gcacatcgct ggtggctgcc cggggcagct      780
gcatcgccaa tgcggaagag gtggatgtac ccatcaagct ctactgtaac ggggacggcg     840
agtggctggt gcccatcggg cgctgcatgt gcaaagcagg cttcgaggcc gttgagaatg     900
gcaccgtctg ccgaggttgt ccatctggga ctttcaaggc caaccaaggg gatgaggcct     960
gtacccactg tcccatcaac agccggacca cttctgaagg ggccaccaac tgtgtctgcc    1020
gcaatggcta ctacagagca gacctggacc ccctggacat gccctgcaca accatcccct    1080
ccgcgcccca ggctgtgatt tccagtgtca atgagacctc cctcatgctg gagtggaccc    1140
ctccccgcga ctccggaggc cgagaggacc tcgtctacaa catcatctgc aagagctgtg    1200
gctcgggccg ggtgcctgc acccgctgcg gggacaatgt acagtacgca ccacgccagc     1260
taggcctgac cgagccacgc atttacatca gtgacctgct ggcccacacc cagtacacct    1320
tcgagatcca ggctgtgaac ggcgttactg accagagccc cttctcgcct cagttcgcct    1380
ctgtgaacat caccaccaac caggcagctc catcggcagt gtccatcatg catcaggtga    1440
gccgcaccgt ggacagcatt accctgtcgt ggtcccagcc agaccagccc aatggcgtga    1500
tcctggacta tgagctgcag tactatgaga aggagctcag tgagtacaac gccacagcca    1560
taaaaagccc caccaacacg gtcaccgtgc agggcctcaa agccggcgcc atctatgtct    1620
tccaggtgcg ggcacgcacc gtggcaggct acgggcgcta cagcggcaag atgtacttcc    1680
agaccatgac agaagccgag taccagacaa gcatccagga gaagttgcca ctcatcatcg    1740
gctcctcggc cgctggcctg gtcttcctca ttgctgtggt tgtcatcgcc atcgtgtgta    1800
acagaagacg gggggtttgag cgtgctgact cggagtacac ggacaagctg caacactaca    1860
ccagtggcca catgacccca ggcatgaaga tctacatcga tcctttcacc tacgaggacc    1920
ccaacgaggc agtgcgggag tttgccaagg aaattgacat ctcctgtgtc aaaattgagc    1980
aggtgatcgg agcaggggag tttggcgagg tctgcagtgg ccacctgaag ctgccaggca    2040
agagagagat ctttgtggcc atcaagacgc tcaagtcggg ctacacggag aagcagcgcc    2100
gggacttcct gagcgaagcc tccatcatgg gccagttcga ccatcccaac gtcatccacc    2160
tggagggtgt cgtgaccaag agcacacctg tgatgatcat caccgagttc atggagaatg    2220
gctccctgga ctcctttctc cggcaaaacg atgggcagtt cacagtcatc cagctggtgg    2280
gcatgcttcg ggcatcgca gctggcatga agtacctggc agacatgaac tatgttcacc     2340
gtgacctggc tgcccgcaac atcctcgtca acagcaacct ggtctgcaag gtgtcggact    2400
ttgggctctc acgctttcta gaggacgata cctcagaccc cacctacacc agtgccctgg    2460
gcggaaagat ccccatccgc tggacagccc cggaagccat ccagtaccgg aagttcacct    2520
cggccagtga tgtgtggagc tacggcattg tcatgtggga ggtgatgtcc tatggggagc    2580
ggccctactg ggacatgacc aaccaggatg taatcaatgc cattgagcag gactatcggc    2640
tgccaccgcc catggactgc ccgagcgccc tgcaccaact catgctggac tgttggcaga    2700
aggaccgcaa ccaccggccc aagttcggcc aaattgtcaa cacgctagac aagatgatcc    2760
gcaatcccaa cagcctcaaa gccatggcgc cctctcctc tggcatcaac ctgccgctgc     2820
tggaccgcac gatccccgac tacaccagct ttaacacggt ggacgagtgg ctggaggcca    2880
```

tcaagatggg gcagtacaag gagagcttcg ccaatgccgg cttcacctcc tttgacgtcg 2940
tgtctcagat gatgatggag gacattctcc gggttgggct cactttggct ggccaccaga 3000
aaaaaatcct gaacagtatc caggtgatgc gggcgcagat gaaccagatt cagtctgtgg 3060
aggtttgaca ttcacctgcc tcggctcacc tcttcctcca agccccgccc cctctgcccc 3120
acgtgccggc cctcctggtg ctctatccac tgcagggcca gccactcgcc aggaggccac 3180
gggccacggg aagaaccaag cggtgccagc cacgagacgt caccaagaaa acatgcaact 3240
caaacgacgg aaaaaaaaag ggaatgggaa aaaagaaaac agatcctggg agggggcggg 3300
aaatacaagg aatatttttt aaagaggatt ctcataagga aagcaatgac tgttcttgcg 3360
ggggataaaa aagggcttgg gagattcatg cgatgtgtcc aatcggagac aaaagcagtt 3420
tctctccaac tccctctggg aaggtgacct ggccagagcc aagaaacact ttcagaaaaa 3480
caaatgtgaa ggggagagac aggggccgcc cttggctcct gtccctgctg ctcctctagg 3540
cctcactcaa caaccaagcg cctggaggac gggacagatg gacagacagc caccctgaga 3600
acccctctgg gaaaatctat tcctgccacc actgggcaaa cagaagaatt tttctgtctt 3660
tggagagtat tttagaaact ccaatgaaag acactgtttc tcctgttggc tcacagggct 3720
gaaaggggct tttgtcctcc tgggtcaggg agaacgcggg gaccccag 3768

<210> 2
<211> 986
<212> PRT
<213> homo sapiens

<400> 2
Met Ala Leu Arg Arg Leu Gly Ala Ala Leu Leu Leu Leu Pro Leu Leu
1 5 10 15
Ala Ala Val Glu Glu Thr Leu Met Asp Ser Thr Thr Ala Thr Ala Glu
20 25 30
Leu Gly Trp Met Val His Pro Pro Ser Gly Trp Glu Glu Val Ser Gly
35 40 45
Tyr Asp Glu Asn Met Asn Thr Ile Arg Thr Tyr Gln Val Cys Asn Val
50 55 60
Phe Glu Ser Ser Gln Asn Asn Trp Leu Arg Thr Lys Phe Ile Arg Arg
65 70 75 80
Arg Gly Ala His Arg Ile His Val Glu Met Lys Phe Ser Val Arg Asp
85 90 95
Cys Ser Ser Ile Pro Ser Val Pro Gly Ser Cys Lys Glu Thr Phe Asn
100 105 110
Leu Tyr Tyr Tyr Glu Ala Asp Phe Asp Ser Ala Thr Lys Thr Phe Pro
115 120 125
Asn Trp Met Glu Asn Pro Trp Val Lys Val Asp Thr Ile Ala Ala Asp
130 135 140
Glu Ser Phe Ser Gln Val Asp Leu Gly Gly Arg Val Met Lys Ile Asn
145 150 155 160
Thr Glu Val Arg Ser Phe Gly Pro Val Ser Arg Ser Gly Phe Tyr Leu
165 170 175
Ala Phe Gln Asp Tyr Gly Gly Cys Met Ser Leu Ile Ala Val Arg Val
180 185 190
Phe Tyr Arg Lys Cys Pro Arg Ile Ile Gln Asn Gly Ala Ile Phe Gln
195 200 205
Glu Thr Leu Ser Gly Ala Glu Ser Thr Ser Leu Val Ala Ala Arg Gly
210 215 220
Ser Cys Ile Ala Asn Ala Glu Glu Val Asp Val Pro Ile Lys Leu Tyr
225 230 235 240
Cys Asn Gly Asp Gly Glu Trp Leu Val Pro Ile Gly Arg Cys Met Cys
245 250 255
Lys Ala Gly Phe Glu Ala Val Glu Asn Gly Thr Val Cys Arg Gly Cys
260 265 270
Pro Ser Gly Thr Phe Lys Ala Asn Gln Gly Asp Glu Ala Cys Thr His
275 280 285
Cys Pro Ile Asn Ser Arg Thr Thr Ser Glu Gly Ala Thr Asn Cys Val
290 295 300
Cys Arg Asn Gly Tyr Tyr Arg Ala Asp Leu Asp Pro Leu Asp Met Pro
305 310 315 320
Cys Thr Thr Ile Pro Ser Ala Pro Gln Ala Val Ile Ser Ser Val Asn
325 330 335
Glu Thr Ser Leu Met Leu Glu Trp Thr Pro Pro Arg Asp Ser Gly Gly
340 345 350
Arg Glu Asp Leu Val Tyr Asn Ile Ile Cys Lys Ser Cys Gly Ser Gly
355 360 365

Arg Gly Ala Cys Thr Arg Cys Gly Asp Asn Val Gln Tyr Ala Pro Arg
370 375 380
Gln Leu Gly Leu Thr Glu Pro Arg Ile Tyr Ile Ser Asp Leu Leu Ala
385 390 395 400
His Thr Gln Tyr Thr Phe Glu Ile Gln Ala Val Asn Gly Val Thr Asp
405 410 415
Gln Ser Pro Phe Ser Pro Gln Phe Ala Ser Val Asn Ile Thr Thr Asn
420 425 430
Gln Ala Ala Pro Ser Ala Val Ser Ile Met His Gln Val Ser Arg Thr
435 440 445
Val Asp Ser Ile Thr Leu Ser Trp Ser Gln Pro Asp Gln Pro Asn Gly
450 455 460
Val Ile Leu Asp Tyr Glu Leu Gln Tyr Tyr Glu Lys Glu Leu Ser Glu
465 470 475 480
Tyr Asn Ala Thr Ala Ile Lys Ser Pro Thr Asn Thr Val Thr Val Gln
485 490 495
Gly Leu Lys Ala Gly Ala Ile Tyr Val Phe Gln Val Arg Ala Arg Thr
500 505 510
Val Ala Gly Tyr Gly Arg Tyr Ser Gly Lys Met Tyr Phe Gln Thr Met
515 520 525
Thr Glu Ala Glu Tyr Gln Thr Ser Ile Gln Glu Lys Leu Pro Leu Ile
530 535 540
Ile Gly Ser Ser Ala Ala Gly Leu Val Phe Leu Ile Ala Val Val Val
545 550 555 560
Ile Ala Ile Val Cys Asn Arg Arg Gly Phe Glu Arg Ala Asp Ser Glu
565 570 575
Tyr Thr Asp Lys Leu Gln His Tyr Thr Ser Gly His Met Thr Pro Gly
580 585 590
Met Lys Ile Tyr Ile Asp Pro Phe Thr Tyr Glu Asp Pro Asn Glu Ala
595 600 605
Val Arg Glu Phe Ala Lys Glu Ile Asp Ile Ser Cys Val Lys Ile Glu
610 615 620
Gln Val Ile Gly Ala Gly Glu Phe Gly Glu Val Cys Ser Gly His Leu
625 630 635 640
Lys Leu Pro Gly Lys Arg Glu Ile Phe Val Ala Ile Lys Thr Leu Lys
645 650 655
Ser Gly Tyr Thr Glu Lys Gln Arg Arg Asp Phe Leu Ser Glu Ala Ser
660 665 670
Ile Met Gly Gln Phe Asp His Pro Asn Val Ile His Leu Glu Gly Val
675 680 685
Val Thr Lys Ser Thr Pro Val Met Ile Ile Thr Glu Phe Met Glu Asn
690 695 700
Gly Ser Leu Asp Ser Phe Leu Arg Gln Asn Asp Gly Gln Phe Thr Val
705 710 715 720
Ile Gln Leu Val Gly Met Leu Arg Gly Ile Ala Ala Gly Met Lys Tyr
725 730 735
Leu Ala Asp Met Asn Tyr Val His Arg Asp Leu Ala Ala Arg Asn Ile
740 745 750
Leu Val Asn Ser Asn Leu Val Cys Lys Val Ser Asp Phe Gly Leu Ser
755 760 765
Arg Phe Leu Glu Asp Asp Thr Ser Asp Pro Thr Tyr Thr Ser Ala Leu
770 775 780
Gly Gly Lys Ile Pro Ile Arg Trp Thr Ala Pro Glu Ala Ile Gln Tyr
785 790 795 800
Arg Lys Phe Thr Ser Ala Ser Asp Val Trp Ser Tyr Gly Ile Val Met
805 810 815
Trp Glu Val Met Ser Tyr Gly Glu Arg Pro Tyr Trp Asp Met Thr Asn
820 825 830
Gln Asp Val Ile Asn Ala Ile Glu Gln Asp Tyr Arg Leu Pro Pro Pro
835 840 845
Met Asp Cys Pro Ser Ala Leu His Gln Leu Met Leu Asp Cys Trp Gln
850 855 860
Lys Asp Arg Asn His Arg Pro Lys Phe Gly Gln Ile Val Asn Thr Leu
865 870 875 880
Asp Lys Met Ile Arg Asn Pro Asn Ser Leu Lys Ala Met Ala Pro Leu
885 890 895
Ser Ser Gly Ile Asn Leu Pro Leu Leu Asp Arg Thr Ile Pro Asp Tyr
900 905 910

Thr Ser Phe Asn Thr Val Asp Glu Trp Leu Glu Ala Ile Lys Met Gly
915 920 925
Gln Tyr Lys Glu Ser Phe Ala Asn Ala Gly Phe Thr Ser Phe Asp Val
930 935 940
Val Ser Gln Met Met Met Glu Asp Ile Leu Arg Leu Gly Val Thr Leu
945 950 955 960
Ala Gly His Gln Lys Lys Ile Leu Asn Ser Ile Gln Val Met Arg Ala
965 970 975
Gln Met Asn Gln Ile Gln Ser Val Glu Val
980 985

<210> 3
<211> 3949
<212> DNA
<213> homo sapiens

<400> 3
gaattccgcc ccgggaagcg cagccatggc tctgcggagg ctgggggccg cgctgctgct 60
gctgccgctg ctcgccgccg tggaagaaac gctaatggac tccactacag cgactgctga 120
gctgggctgg atggtgcatc ctccatcagg gtgggaagag gtgagtggct acgatgagaa 180
catgaacacg atccgcacgt accaggtgtg caacgtgttt gagtcaagcc agaacaactg 240
gctacggacc aagtttatcc ggcgccgtgg cgcccaccgc atccacgtgg agatgaagtt 300
ttcggtgcgt gactgcagca gcatccccag cgtgcctggc tcctgcaagg agaccttcaa 360
cctctattac tatgaggctg actttgactc ggccaccaag accttcccca actggatgga 420
gaatccatgg gtgaaggtgg ataccattgc agccgacgag agcttctccc aggtggacct 480
gggtggccgc gtcatgaaaa tcaacaccga ggtgcggagc ttcggacctg tgtcccgcag 540
cggcttctac ctggccttcc aggactatgg cggctgcatg tccctcatcg ccgtgcgtgt 600
cttctaccgc aagtgccccc gcatcatcca gaatggcgcc atcttccagg aaaccctgtc 660
gggggctgag agcacatcgc tggtggctgc ccggggcagc tgcatcgcca atgcggaaga 720
ggtggatgta cccatcaagc tctactgtaa cggggacggc gagtggctgg tgcccatcgg 780
gcgctgcatg tgcaaagcag gcttcgaggc cgttgagaat ggcaccgtct gccgaggttg 840
tccatctggg actttcaagg ccaaccaagg ggatgaggcc tgtacccact gtcccatcaa 900
cagccggacc acttctgaag ggccaccaa ctgtgtctgc cgcaatggct actacagagc 960
agacctggac cccctggaca tgccctgcac aaccatcccc tccgcgcccc aggctgtgat 1020
ttccagtgtc aatgagacct ccctcatgct ggagtggacc cctccccgcg actccggagg 1080
ccgagaggac ctcgtctaca acatcatctg caagagctgt ggctcgggcc ggggtgcctg 1140
caccccgctgc ggggacaatg tacagtacgc accacgccag ctaggcctga ccgagccacg 1200
catttacatc agtgacctgc tggcccacac ccagtacacc ttcgagatcc aggctgtgaa 1260
cggcgttact gaccagagcc ccttctcgcc tcagttcgcc tctgtgaaca tcaccaccaa 1320
ccaggcagct ccatcggcag tgtccatcat gcatcaggtg agccgcaccg tggacagcat 1380
taccctgtcg tggtcccagc cagaccagcc caatggcgtg atcctggact atgagctgca 1440
gtactatgag aaggagctca gtgagtacaa cgccacagcc ataaaaagcc ccaccaacac 1500
ggtcaccgtg cagggcctca aagccggcgc catctatgtc ttccaggtgc gggcacgcac 1560
cgtggcaggc tacgggcgct acagcggcaa gatgtacttc cagaccatga cagaagccga 1620
gtaccagaca agcatccagg agaagttgcc actcatcatc ggctcctcgg ccgctggcct 1680
ggtcttcctc attgctgtgg ttgtcatcgc catcgtgtgt aacagacggg ggtttgagcg 1740
tgctgactcg gagtacacgg acaagctgca acactacacc agtggccaca tgaccccagg 1800
catgaagatc tacatcgatc ctttcaccta cgaggacccc aacgaggcag tgcgggagtt 1860
tgccaaggaa attgacatct cctgtgtcaa aattgagcag gtgatcggag caggggagtt 1920
tggcgaggtc tgcagtggcc acctgaagct gccaggcaag agagagatct ttgtggccat 1980
caagacgctc aagtcgggct acacggagaa gcagcgccgg gacttcctga gcgaagcctc 2040
catcatgggc cagttcgacc atcccaacgt catccacctg gagggtgtcg tgaccaagag 2100
cacacctgtg atgatcatca ccgagttcat ggagaatggc tccctggact cctttctccg 2160
gcaaaacgat gggcagttca cagtcatcca gctggtgggc atgcttcggg gcatcgcagc 2220
tggcatgaag tacctggcag acatgaacta tgttcaccgt gacctggctg cccgcaacat 2280
cctcgtcaac agcaacctgg tctgcaaggt gtcggacttt gggctctcac gctttctaga 2340
ggacgatacc tcagacccca cctacaccag tgccctgggc ggaaagatcc ccatccgctg 2400
gacagccccg gaagccatcc agtaccggaa gttcacctcg gccagtgatg tgtggagcta 2460
cggcattgtc atgtgggagg tgatgtccta tggggagcgg ccctactggg acatgaccaa 2520
ccaggatgta atcaatgcca ttgagcagga ctatcggctg ccaccgccca tggactgccc 2580
gagcgccctg caccaactca tgctggactg ttggcagaag gaccgcaacc accggcccaa 2640
gttcggccaa attgtcaaca cgctagacaa gatgatccgc aatcccaaca gcctcaaagc 2700
catggcgccc ctctcctctg gcatcaacct gccgctgctg gaccgcacga tccccgacta 2760
caccagcttt aacacggtgg acgagtggct ggaggccatc aagatggggc agtacaagga 2820
gagcttcgcc aatgccggct tcacctcctt tgacgtcgtg tctcagatga tgatggagga 2880
cattctccgg gttggggtca ctttggctgg ccaccagaaa aaaatcctga acagtatcca 2940
ggtgatgcgg gcgcagatga accagattca gtctgtggag ggccagccac tcgccaggag 3000
gccacgggcc acgggaagaa ccaagcggtg ccagccacga gacgtcacca agaaaacatg 3060

caactcaaac gacggaaaaa aaaagggaat gggaaaaaag aaaacagatc ctgggagggg 3120
gcgggaaata caaggaatat tttttaaaga ggattctcat aaggaaagca atgactgttc 3180
ttgcggggga taaaaaaggg cttgggagat tcatgcgatg tgtccaatcg gagacaaaag 3240
cagtttctct ccaactccct ctgggaaggt gacctggcca gagccaagaa acactttcag 3300
aaaaacaaat gtgaagggga gagacagggg ccacccttgg ctcctgtccc tgctgctcct 3360
ctaggcctca ctcaacaacc aagcgcctgg aggacgggac agatggacag acagccaccc 3420
tgagaacccc tctgggaaaa tctattcctg ccaccactgg gcaaacagaa gaatttttct 3480
gtctttggag agtattttag aaactccaat gaaagacact gtttctcctg ttggctcaca 3540
gggctgaaag gggcttttgt cctcctgggt cagggagaac gcggggaccc cagaaaggtc 3600
agccttcctg aggatgggca acccccaggt ctgcagctcc aggtacatat cacgcgcaca 3660
gcctggcagc ctggccctcc tggtgcccac tcccgccagc ccctgcctcg aggactgata 3720
ctgcagtgac tgccgtcagc tccgactgcc gctgagaagg gttgatcctg catctgggtt 3780
tgtttacagc aattcctgga ctcgggggta ttttggtcac agggtggttt tggtttaggg 3840
ggtttgtttg ttgggttgtt ttttgttttt tggttttttt taatgacaat gaagtgacac 3900
tttgacattt ccaaaaaaaa aaaaaaaaaa aaaaaaaaaa aaaaaaaaa 3949

<210> 4
<211> 1055
<212> PRT
<213> homo sapiens

<400> 4
Met Ala Leu Arg Arg Leu Gly Ala Ala Leu Leu Leu Leu Pro Leu Leu
1 5 10 15
Ala Ala Val Glu Glu Thr Leu Met Asp Ser Thr Thr Ala Thr Ala Glu
20 25 30
Leu Gly Trp Met Val His Pro Pro Ser Gly Trp Glu Glu Val Ser Gly
35 40 45
Tyr Asp Glu Asn Met Asn Thr Ile Arg Thr Tyr Gln Val Cys Asn Val
50 55 60
Phe Glu Ser Ser Gln Asn Asn Trp Leu Arg Thr Lys Phe Ile Arg Arg
65 70 75 80
Arg Gly Ala His Arg Ile His Val Glu Met Lys Phe Ser Val Arg Asp
85 90 95
Cys Ser Ser Ile Pro Ser Val Pro Gly Ser Cys Lys Glu Thr Phe Asn
100 105 110
Leu Tyr Tyr Tyr Glu Ala Asp Phe Asp Ser Ala Thr Lys Thr Phe Pro
115 120 125
Asn Trp Met Glu Asn Pro Trp Val Lys Val Asp Thr Ile Ala Ala Asp
130 135 140
Glu Ser Phe Ser Gln Val Asp Leu Gly Gly Arg Val Met Lys Ile Asn
145 150 155 160
Thr Glu Val Arg Ser Phe Gly Pro Val Ser Arg Ser Gly Phe Tyr Leu
165 170 175
Ala Phe Gln Asp Tyr Gly Gly Cys Met Ser Leu Ile Ala Val Arg Val
180 185 190
Phe Tyr Arg Lys Cys Pro Arg Ile Ile Gln Asn Gly Ala Ile Phe Gln
195 200 205
Glu Thr Leu Ser Gly Ala Glu Ser Thr Ser Leu Val Ala Ala Arg Gly
210 215 220
Ser Cys Ile Ala Asn Ala Glu Glu Val Asp Val Pro Ile Lys Leu Tyr
225 230 235 240
Cys Asn Gly Asp Gly Glu Trp Leu Val Pro Ile Gly Arg Cys Met Cys
245 250 255
Lys Ala Gly Phe Glu Ala Val Glu Asn Gly Thr Val Cys Arg Gly Cys
260 265 270
Pro Ser Gly Thr Phe Lys Ala Asn Gln Gly Asp Glu Ala Cys Thr His
275 280 285
Cys Pro Ile Asn Ser Arg Thr Thr Ser Glu Gly Ala Thr Asn Cys Val
290 295 300
Cys Arg Asn Gly Tyr Tyr Arg Ala Asp Leu Asp Pro Leu Asp Met Pro
305 310 315 320
Cys Thr Thr Ile Pro Ser Ala Pro Gln Ala Val Ile Ser Ser Val Asn
325 330 335
Glu Thr Ser Leu Met Leu Glu Trp Thr Pro Pro Arg Asp Ser Gly Gly
340 345 350
Arg Glu Asp Leu Val Tyr Asn Ile Ile Cys Lys Ser Cys Gly Ser Gly
355 360 365

```
Arg Gly Ala Cys Thr Arg Cys Gly Asp Asn Val Gln Tyr Ala Pro Arg
    370                 375                 380
Gln Leu Gly Leu Thr Glu Pro Arg Ile Tyr Ile Ser Asp Leu Leu Ala
385                 390                 395                 400
His Thr Gln Tyr Thr Phe Glu Ile Gln Ala Val Asn Gly Val Thr Asp
                405                 410                 415
Gln Ser Pro Phe Ser Pro Gln Phe Ala Ser Val Asn Ile Thr Thr Asn
            420                 425                 430
Gln Ala Ala Pro Ser Ala Val Ser Ile Met His Gln Val Ser Arg Thr
        435                 440                 445
Val Asp Ser Ile Thr Leu Ser Trp Ser Gln Pro Asp Gln Pro Asn Gly
    450                 455                 460
Val Ile Leu Asp Tyr Glu Leu Gln Tyr Tyr Glu Lys Glu Leu Ser Glu
465                 470                 475                 480
Tyr Asn Ala Thr Ala Ile Lys Ser Pro Thr Asn Thr Val Thr Val Gln
                485                 490                 495
Gly Leu Lys Ala Gly Ala Ile Tyr Val Phe Gln Val Arg Ala Arg Thr
            500                 505                 510
Val Ala Gly Tyr Gly Arg Tyr Ser Gly Lys Met Tyr Phe Gln Thr Met
        515                 520                 525
Thr Glu Ala Glu Tyr Gln Thr Ser Ile Gln Glu Lys Leu Pro Leu Ile
    530                 535                 540
Ile Gly Ser Ser Ala Ala Gly Leu Val Phe Leu Ile Ala Val Val Val
545                 550                 555                 560
Ile Ala Ile Val Cys Asn Arg Arg Gly Phe Glu Arg Ala Asp Ser Glu
                565                 570                 575
Tyr Thr Asp Lys Leu Gln His Tyr Thr Ser Gly His Met Thr Pro Gly
            580                 585                 590
Met Lys Ile Tyr Ile Asp Pro Phe Thr Tyr Glu Asp Pro Asn Glu Ala
        595                 600                 605
Val Arg Glu Phe Ala Lys Glu Ile Asp Ile Ser Cys Val Lys Ile Glu
    610                 615                 620
Gln Val Ile Gly Ala Gly Glu Phe Gly Glu Val Cys Ser Gly His Leu
625                 630                 635                 640
Lys Leu Pro Gly Lys Arg Glu Ile Phe Val Ala Ile Lys Thr Leu Lys
                645                 650                 655
Ser Gly Tyr Thr Glu Lys Gln Arg Arg Asp Phe Leu Ser Glu Ala Ser
            660                 665                 670
Ile Met Gly Gln Phe Asp His Pro Asn Val Ile His Leu Glu Gly Val
        675                 680                 685
Val Thr Lys Ser Thr Pro Val Met Ile Ile Thr Glu Phe Met Glu Asn
    690                 695                 700
Gly Ser Leu Asp Ser Phe Leu Arg Gln Asn Asp Gly Gln Phe Thr Val
705                 710                 715                 720
Ile Gln Leu Val Gly Met Leu Arg Gly Ile Ala Ala Gly Met Lys Tyr
                725                 730                 735
Leu Ala Asp Met Asn Tyr Val His Arg Asp Leu Ala Ala Arg Asn Ile
            740                 745                 750
Leu Val Asn Ser Asn Leu Val Cys Lys Val Ser Asp Phe Gly Leu Ser
        755                 760                 765
Arg Phe Leu Glu Asp Asp Thr Ser Asp Pro Thr Tyr Thr Ser Ala Leu
    770                 775                 780
Gly Gly Lys Ile Pro Ile Arg Trp Thr Ala Pro Glu Ala Ile Gln Tyr
785                 790                 795                 800
Arg Lys Phe Thr Ser Ala Ser Asp Val Trp Ser Tyr Gly Ile Val Met
                805                 810                 815
Trp Glu Val Met Ser Tyr Gly Glu Arg Pro Tyr Trp Asp Met Thr Asn
            820                 825                 830
Gln Asp Val Ile Asn Ala Ile Glu Gln Asp Tyr Arg Leu Pro Pro Pro
        835                 840                 845
Met Asp Cys Pro Ser Ala Leu His Gln Leu Met Leu Asp Cys Trp Gln
    850                 855                 860
Lys Asp Arg Asn His Arg Pro Lys Phe Gly Gln Ile Val Asn Thr Leu
865                 870                 875                 880
Asp Lys Met Ile Arg Asn Pro Asn Ser Leu Lys Ala Met Ala Pro Leu
                885                 890                 895
Ser Ser Gly Ile Asn Leu Pro Leu Leu Asp Arg Thr Ile Pro Asp Tyr
            900                 905                 910
```

Thr Ser Phe Asn Thr Val Asp Glu Trp Leu Glu Ala Ile Lys Met Gly
915 920 925
Gln Tyr Lys Glu Ser Phe Ala Asn Ala Gly Phe Thr Ser Phe Asp Val
930 935 940
Val Ser Gln Met Met Met Glu Asp Ile Leu Arg Val Gly Val Thr Leu
945 950 955 960
Ala Gly His Gln Lys Lys Ile Leu Asn Ser Ile Gln Val Met Arg Ala
965 970 975
Gln Met Asn Gln Ile Gln Ser Val Glu Gly Gln Pro Leu Ala Arg Arg
980 985 990
Pro Arg Ala Thr Gly Arg Thr Lys Arg Cys Gln Pro Arg Asp Val Thr
995 1000 1005
Lys Lys Thr Cys Asn Ser Asn Asp Gly Lys Lys Lys Gly Met Gly Lys
1010 1015 1020
Lys Lys Thr Asp Pro Gly Arg Gly Arg Glu Ile Gln Gly Ile Phe Phe
1025 1030 1035 104
Lys Glu Asp Ser His Lys Glu Ser Asn Asp Cys Ser Cys Gly Gly
1045 1050 1055

<210> 5
<211> 9
<212> PRT
<213> Artificial Sequence

<400> 5
Met Met Met Glu Asp Ile Leu Arg Val
1 5

<210> 6
<211> 9
<212> PRT
<213> Artificial Sequence

<400> 6
Leu Leu Leu Pro Leu Leu Ala Ala Val
1 5

<210> 7
<211> 9
<212> PRT
<213> Artificial Sequence

<400> 7
Gly Leu Thr Glu Pro Arg Ile Tyr Ile
1 5

<210> 8
<211> 9
<212> PRT
<213> Artificial Sequence

<400> 8
Arg Gln Asn Asp Gly Gln Phe Thr Val
1 5

<210> 9
<211> 9
<212> PRT
<213> Artificial Sequence

<400> 9
Thr Leu Met Asp Ser Thr Thr Ala Thr
1 5

<210> 10
<211> 9
<212> PRT

```
     <213> Artificial Sequence

     <400> 10
Lys Leu Pro Gly Arg Glu Ile Phe Val
 1               5

     <210> 11
     <211> 9
     <212> PRT
     <213> Artificial Sequence

     <400> 11
Leu Leu Leu Leu Leu Leu Ala Ala Val
 1               5

     <210> 12
     <211> 9
     <212> PRT
     <213> Artificial Sequence

     <400> 12
Gln Met Met Met Asp Ile Leu Arg Val
 1               5

     <210> 13
     <211> 9
     <212> PRT
     <213> Artificial Sequence

     <400> 13
Trp Ser Tyr Gly Val Met Trp Glu Val
 1               5

     <210> 14
     <211> 9
     <212> PRT
     <213> Artificial Sequence

     <400> 14
Phe Leu Ile Ala Val Val Ile Ala Ile
 1               5
```